# EUROPEAN PATENT APPLICATION

(11) **EP 2 644 602 A1**
(43) Date of publication of application: **02.10.2013**
(21) Application number: 12161228.7
(22) Date of filing: 26.03.2012
(51) Int. Cl.: C07D 265/06, A23L 1/227, A23L 1/22

(54) **Use of oxazolines as aroma/flavour precursors**

(71) Applicant: Nestec S.A., 1800 Vevey (CH)
(72) Inventor: Blank, Imre, 1073 Savigny (CH); Davidek, Thomas, 1410 Correvon (CH); Novotny, Ondrej, 1012 Lausanne (CH); Schieberle, Peter, 85406 Zolling (DE); Granvogl, Michael, 80997 München (DE)
(74) Representative: Lomholt, Stig Bredsted

(57) **Abstract**

The present invention relates to compounds based on an oxazoline moiety which liberate Strecker aldehydes under mild and controllable conditions. In addition the invention relates to food products comprising such compounds, and uses of such compounds.

## Description

### Technical field of the invention

The present invention relates to oxazoline compounds as precursor molecules for controlled release of Strecker aldehydes. In particular the present invention relates to food products enriched with such oxazoline compounds.

### Background of the invention

Flavour (or aroma) is a quality attribute for food and beverages. However, flavour stability is a known issue and there is a need to develop concepts for flavour stabilization. Flavour stability might be solved by suitable packaging and/or encapsulation systems that prevent aroma compounds from deterioration due to chemical and enzymatic reactions. Depending on the flavour system and matrix environment, these approaches have serious limitations.

Flavour deterioration may happen in a broad time span from minutes to years. It starts with food processing, storage, and the preparation event. The reasons leading to off-notes may also be very different (e.g. oxygen, temperature, moisture, light, etc.). Therefore, preserving desirable aroma is definitely a challenging task.

Complex flavours are composed of many aroma-active compounds. Amongst them, the so-called Strecker aldehydes play a pivotal role such as methylpropanal, 2-methylbutanal, 3-methylbutanal, phyenylacetaldehyde, and methional. In certain food systems, they may have the role of character impact compounds. These compounds are formed through the Strecker reaction in the course of the Maillard reaction cascade. As there are many competing reactions taking place, it is not obvious to find conditions favouring the formation of Strecker aldehydes compared to other compounds of less aroma significance. Thus, there is a need for more control in the aroma formation phase.

Hence, improved precursor flavour/aroma compounds would be advantageous, and in particular a more efficient and/or reliable way of releasing the flavour/aroma compounds would be advantageous.

### Summary of the invention

Generation of authentic fresh aroma prior to or during the consumption event may be an alternative approach to aroma preservation in order to deliver desirable flavour notes. Flavour generation under mild and controlled conditions is a major challenge in the food industry.

Thus, an object of the present invention relates to the provision of compounds which may release flavour and/or aroma compounds under mild and controlled conditions.

The present invention solves the above stated problem by providing precursor compounds based on an oxazoline moiety which liberate Strecker aldehydes under mild and controllable conditions.

Thus, one aspect of the invention relates to a compound of the formula 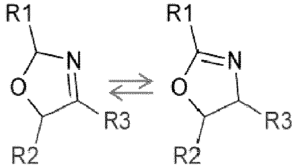 wherein
- R1 is selected from the group consisting of hydrogen, a hydrocarbon, a thiohydrocarbon and an aminohydrocarbon; and
- R2 and R3 are, independent from each other and selected from the group consisting of a hydrogen, a hydrocarbon, a carbonyl, a hydroxycarbonyl, a polyol, and aminohydrocarbon, or R2 is being linked to R3 by a bridge member Yₙ, thereby forming one or more rings; wherein Yₙ is being selected from the group consisting of a bond, C1-12 alkyl, aryl, a carbocyclic moiety, a heterocyclic moiety and a heteroaromatic moiety;
   and wherein the compound is not, 2,5-Dihydro-2-methyl-oxazole, 4,5-Dihydro-2-(1-methylethyl)-oxazole, 4,5-Dihydro-2-(1-methylpropyl)-oxazole, 4,5-Dihydro-2-(2-methylpropyl)-oxazole, 4,5-Dihydro-2-(phenylmethyl)-oxazole, 2,5-Dihydro-2,4-dimethyl-oxazole, 4,5-Dihydro-2,4-dimethyl-oxazole, 2,5-Dihydro-4-methyl-2-(1-methylethyl)-oxazole, 4,5-Dihydro-4-methyl-2-(1-methylethyl)-oxazole, 4,5-Dihydro-4-methyl-2-(phenylmethyl)-oxazole, 2,5-Dihydro-4-methyl-2-(phenylmethyl)-oxazole, 2,5-Dihydro-2,5-dimethyl-oxazole, 4,5-Dihydro-2,5-dimethyl-oxazole, 2,5-Dihydro-5-methyl-2-(1-methylethyl)-oxazole, 4,5-Dihydro-5-methyl-2-(1-methylethyl)-oxazole, 4,5-Dihydro-5-methyl-2-(phenylmethyl)-oxazole, 2,5-Dihydro-2,4,5-trimethyl-oxazole, 4,5-Dihydro-2,4,5-dimethyl-oxazole, 2,5-Dihydro-4,5-dimethyl-2-(1-methylethyl)-oxazole, 2,5-Dihydro-4,5-dimethyl-2-(2-methylpropyl)-oxazole, 4,5-Dihydro-4,5-dimethyl-2-(phenylmethyl)-oxazole, 4-Ethyl-2,5-dihydro-2,5-dimethyl-oxazole, 4-Ethyl-2,5-dihydro-5-methyl-2-(2-methylpropyl)-oxazole, 5-Ethyl-2,5-dihydro-2,4-dimethyl-oxazole, or 5-Ethyl-2,5-dihydro-4-methyl-2-(2-methylpropyl)-oxazole, or 4,5-Dihydro-2-methyl-5-oxazolemethanol.

Another aspect of the present invention relates to a method for obtaining a compound according to the invention comprising
a) chemically synthesizing the compound; or
b) isolating or generating an enriched fraction of the compound from a natural source; or
c) providing the compound by fermentation of a micro-organism.

Yet another aspect of the present invention is to provide a food ingredient enriched with one or more compounds of the formula 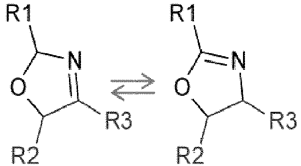 wherein
- R1 is selected from the group consisting of hydrogen, a hydrocarbon, a thiohydrocarbon and an aminohydrocarbon; and
- R2 and R3 are, independent from each other and selected from the group consisting of a hydrogen, a hydrocarbon, a carbonyl, a hydroxycarbonyl, a polyol, and aminohydrocarbon, or R2 is being linked to R3 by a bridge member Yₙ, thereby forming one or more rings; wherein Yₙ is being selected from the group consisting of a bond, C1-12 alkyl, aryl, a carbocyclic moiety, a heterocyclic moiety and a heteroaromatic moiety;
   wherein the compound is not 2-methyl-3-oxazoline, 2,4-dimethyl-3-oxazoline, 2,5-dimethyl-3-oxazoline, 2,4,5-trimethyl-3-oxazoline, 5-ethyl-2,4-dimethyl-3-oxazoline, or 4-ethyl-2,5-dimethyl-3-oxazoline.

Still another aspect of the present invention is to provide a food product enriched with one or more compounds of the formula 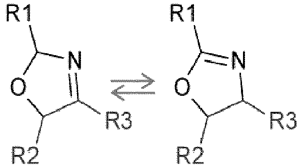 wherein
- R1 is selected from the group consisting of hydrogen, a hydrocarbon, a thiohydrocarbon and an aminohydrocarbon; and
- R2 and R3 are, independent from each other and selected from the group consisting of a hydrogen, a hydrocarbon, a carbonyl, a hydroxycarbonyl, a polyol, and aminohydrocarbon, or R2 is being linked to R3 by a bridge member Yₙ, thereby forming one or more rings; wherein Yₙ is being selected from the group consisting of a bond, C1-12 alkyl, aryl, a carbocyclic moiety, a heterocyclic moiety and a heteroaromatic moiety;
   wherein the compound is not 2-methyl-3-oxazoline, 2,4-dimethyl-3-oxazoline, 2,5-dimethyl-3-oxazoline, 2,4,5-trimethyl-3-oxazoline, 5-ethyl-2,4-dimethyl-3-oxazoline, or 4-ethyl-2,5-dimethyl-3-oxazoline;
   or
   a food product enriched with a food ingredient according to the present invention.

An aspect also relates to a method for producing a flavor/aroma enriched food product or food ingredient comprising
a) providing a food product or food ingredient;
b) providing one or more compounds of the formula 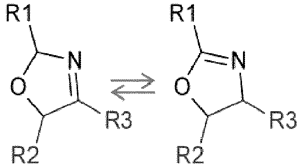 wherein
   o R1 is selected from the group consisting of hydrogen, a hydrocarbon, a thiohydrocarbon and an aminohydrocarbon; and
   o R2 and R3 are, independent from each other and selected from the group consisting of a hydrogen, a hydrocarbon, a carbonyl, a hydroxycarbonyl, a polyol, and aminohydrocarbon, or R2 is being linked to R3 by a bridge member Yₙ, thereby forming one or more rings; wherein Yₙ is being selected from the group consisting of a bond, C1-12 alkyl, aryl, a carbocyclic moiety, a heterocyclic moiety and a heteroaromatic moiety;
   wherein the compound is not 2-methyl-3-oxazoline, 2,4-dimethyl-3-oxazoline, 2,5-dimethyl-3-oxazoline, 2,4,5-trimethyl-3-oxazoline, 5-ethyl-2,4-dimethyl-3-oxazoline, or 4-ethyl-2,5-dimethyl-3-oxazoline according to any of claims 1-3; and
c) mixing a) and b.

A further aspect relates to the use of a compound of the formula 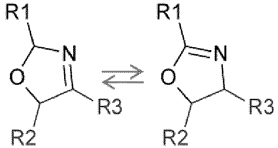 wherein
- R1 is selected from the group consisting of hydrogen, a hydrocarbon, a thiohydrocarbon and an aminohydrocarbon;
- R2 and R3 are, independent from each other and selected from the group consisting of a hydrogen, a hydrocarbon, a carbonyl, a hydroxycarbonyl, a polyol, and aminohydrocarbon, or
- R2 is being linked to R3 by a bridge member Yₙ, thereby forming one or more rings; wherein Yₙ is being selected from the group consisting of a bond, C1-12 alkyl, aryl, a carbocyclic moiety, a heterocyclic moiety and a heteroaromatic moiety,
as a flavor/aroma precursor.

Another aspect relates to the use of a compound of the formula 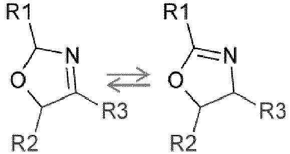 wherein
- R1 is selected from the group consisting of hydrogen, a hydrocarbon, a thiohydrocarbon and an aminohydrocarbon;
- R2 and R3 are, independent from each other and selected from the group consisting of a hydrogen, a hydrocarbon, a carbonyl, a hydroxycarbonyl, a polyol, and aminohydrocarbon, or
- R2 is being linked to R3 by a bridge member Yₙ, thereby forming one or more rings; wherein Yₙ is being selected from the group consisting of a bond, C1-12 alkyl, aryl, a carbocyclic moiety, a heterocyclic moiety and a heteroaromatic moiety,
in a Strecker aldehyde release system.

Still another aspect relates to the use of a food ingredient according to the invention as a flavor/aroma precursor in food products.

Yet an aspect relates to a method for releasing a Strecker aldehyde from a composition having a water activity in the range 0.01-0.7 and comprising one or more compounds of the formula 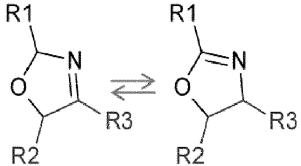 wherein
- R1 is selected from the group consisting of hydrogen, a hydrocarbon, a thiohydrocarbon and an aminohydrocarbon;
- R2 and R3 are, independent from each other and selected from the group consisting of a hydrogen, a hydrocarbon, a carbonyl, a hydroxycarbonyl, a polyol, and aminohydrocarbon, or
- R2 is being linked to R3 by a bridge member Yₙ, thereby forming one or more rings; wherein Yₙ is being selected from the group consisting of a bond, C1-12 alkyl, aryl, a carbocyclic moiety, a heterocyclic moiety and a heteroaromatic moiety,
the method comprising adding an aqueous liquid to the composition.

### Brief description of the figures

### Figure 1

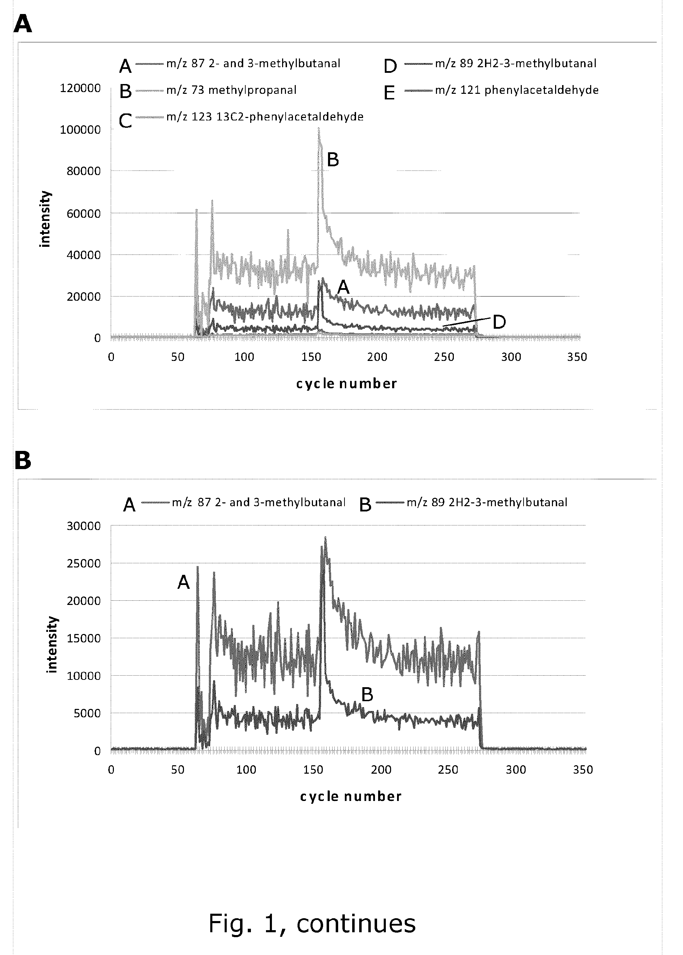

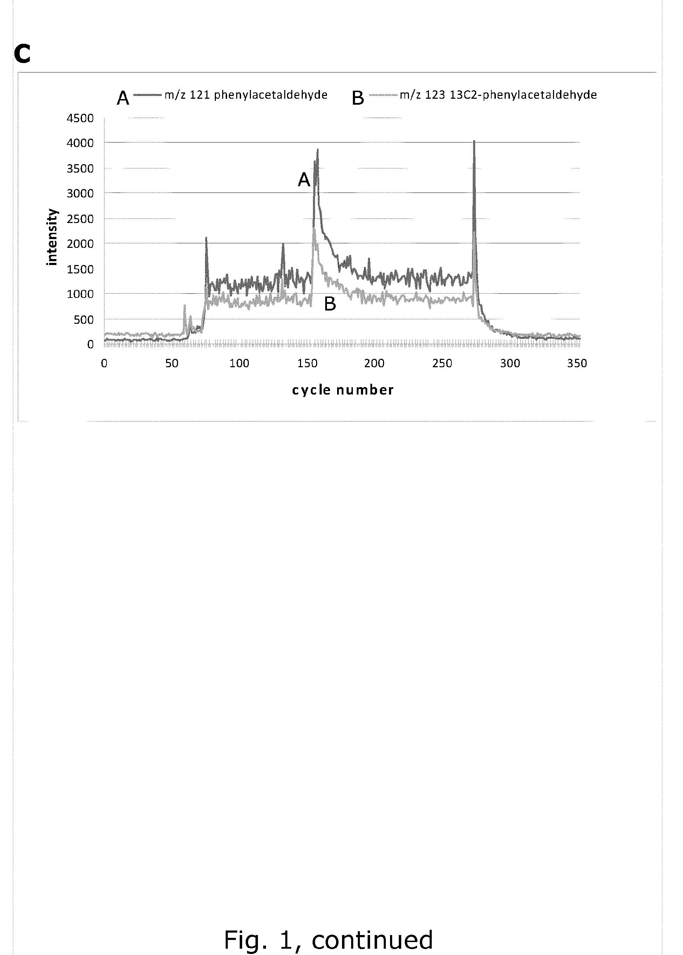

Figure 1 shows addition of water (5 mL; at cycle number: 155) labelled and unlabelled aroma-active compounds in sunflower oil (10 g) at 37°C (reference sample).
A: Methylpropanal, sum of 2- and 3-methylbutanal, phenylacetaldehyde, [²H₂]-3-methylbutanal, and [¹³C₂]-phenylacetaldehyde;
B: Sum of 2- and 3-methylbutanal and [²H₂]-3-methylbutanal;
C: Phenylacetaldehyde and [¹³C₂]-phenylacetaldehyde.

### Figure 2

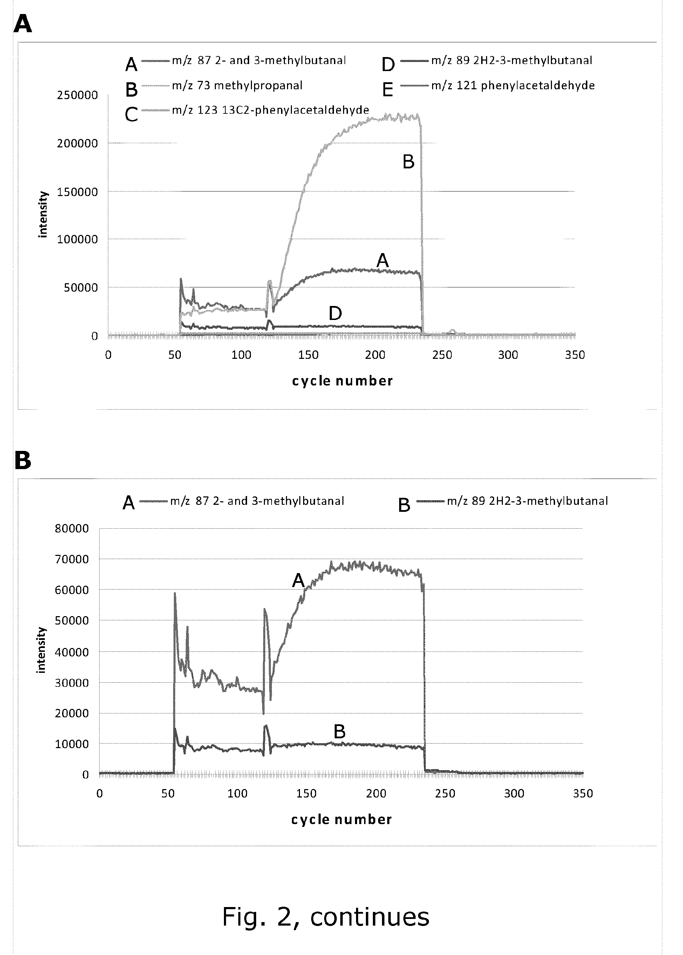

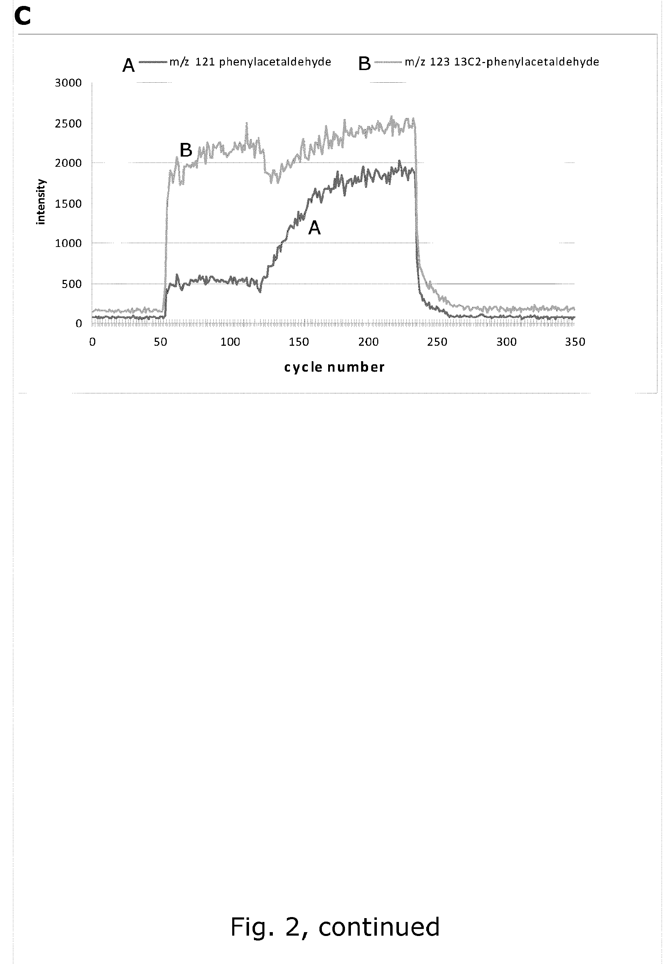

Figure 2 shows addition of water (5 mL; at cycle number: 120) to spiked chocolate (5 g) at 37 °C.
A: Methylpropanal, sum of 2- and 3-methylbutanal, phenylacetaldehyde, [²H₂]-3-methylbutanal, and [¹³C₂]-phenylacetaldehyde
B: Sum of 2- and 3-methylbutanal and [²H₂]-3-methylbutanal
C: Phenylacetaldehyde and [¹³C₂]-phenylacetaldehyde

### Figure 3

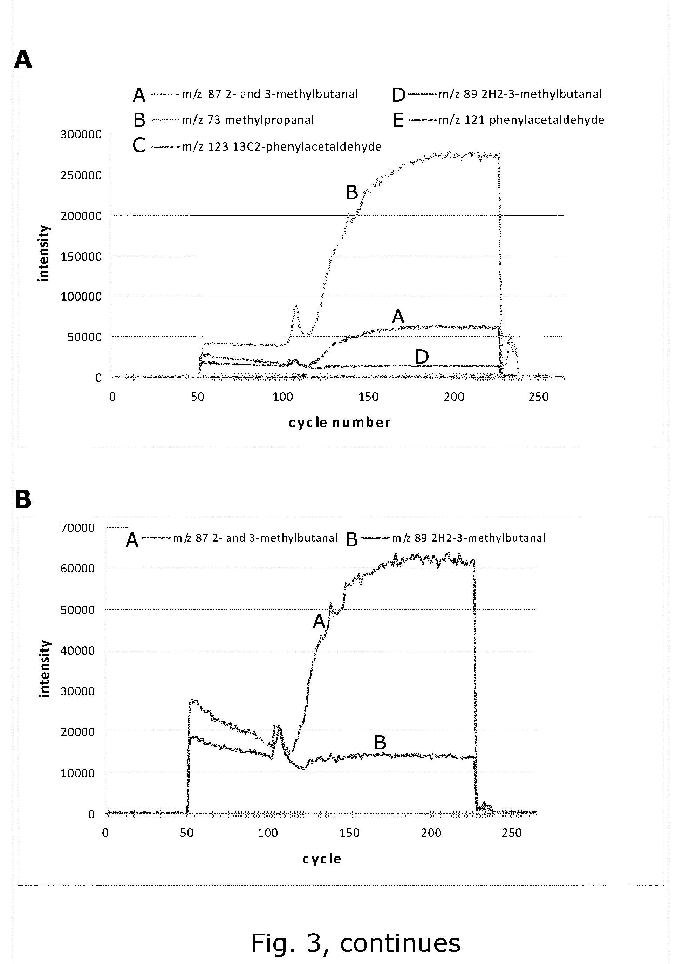

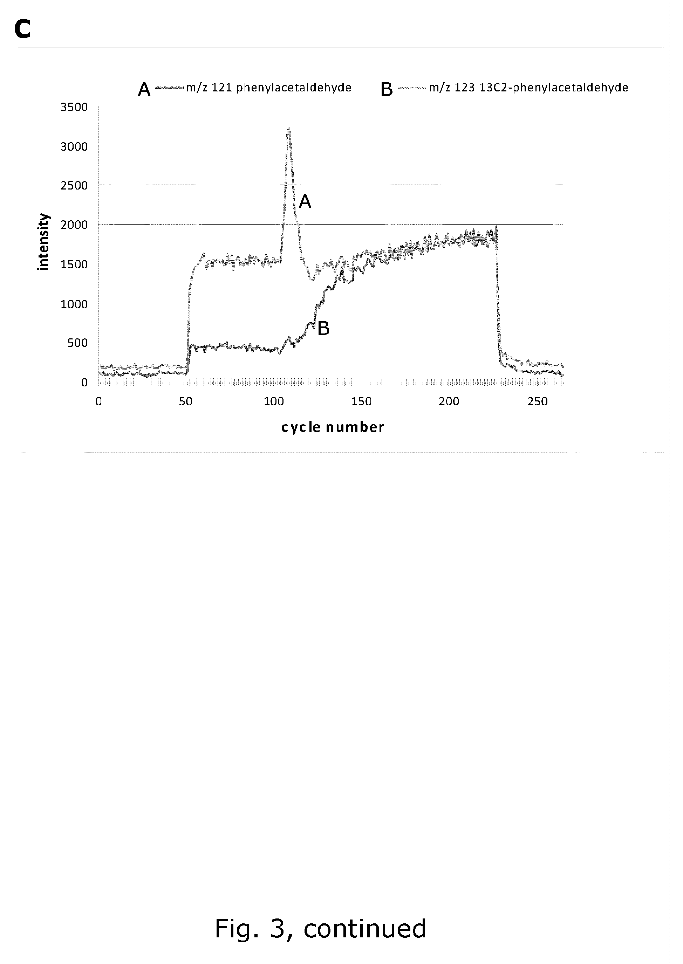

Figure 3 shows addition of saliva (5 mL; at cycle number: 100-104) to spiked chocolate (5 g) at 37 °C.
A: Methylpropanal, sum of 2- and 3-methylbutanal, phenylacetaldehyde, [²H₂]-3-methylbutanal, and [¹³C₂]-phenylacetaldehyde.
B: Sum of 2- and 3-methylbutanal and [²H₂]-3-methylbutanal.
C: Phenylacetaldehyde and [¹³C₂]-phenylacetaldehyde.

### Figure 4

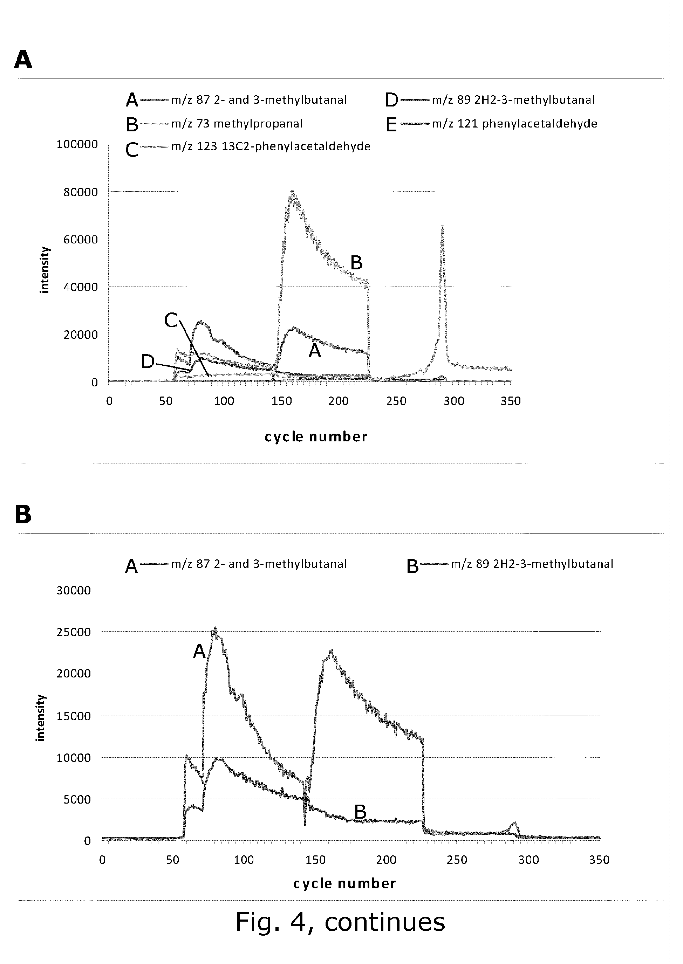

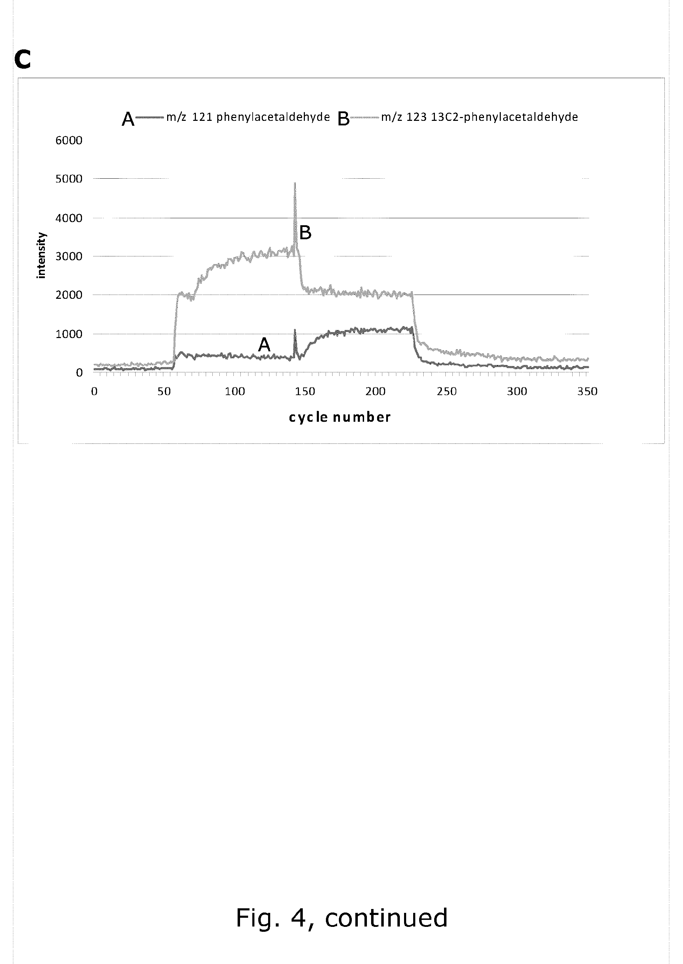

Figure 4 shows the release of 2- and 3-methylbutanal as well as phenylacetaldehyde from spiked chocolate.
A: Methylpropanal, sum of 2- and 3-methylbutanal, phenylacetaldehyde, [²H₂]-3-methylbutanal, and [¹³C₂]-phenylacetaldehyde.
B: Sum of 2- and 3-methylbutanal and [²H₂]-3-methylbutanal.
C: Phenylacetaldehyde and [¹³C₂]-phenylacetaldehyde.

### Figure 5

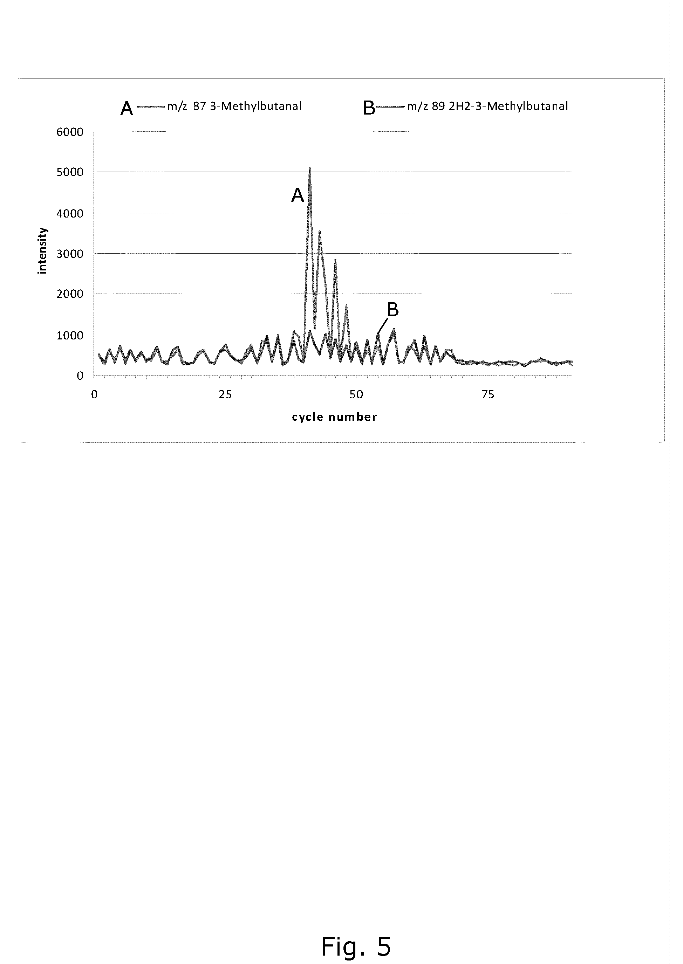

Figure 5 shows the release of 3-methylbutanal from spiked chocolate.

### Figure 6

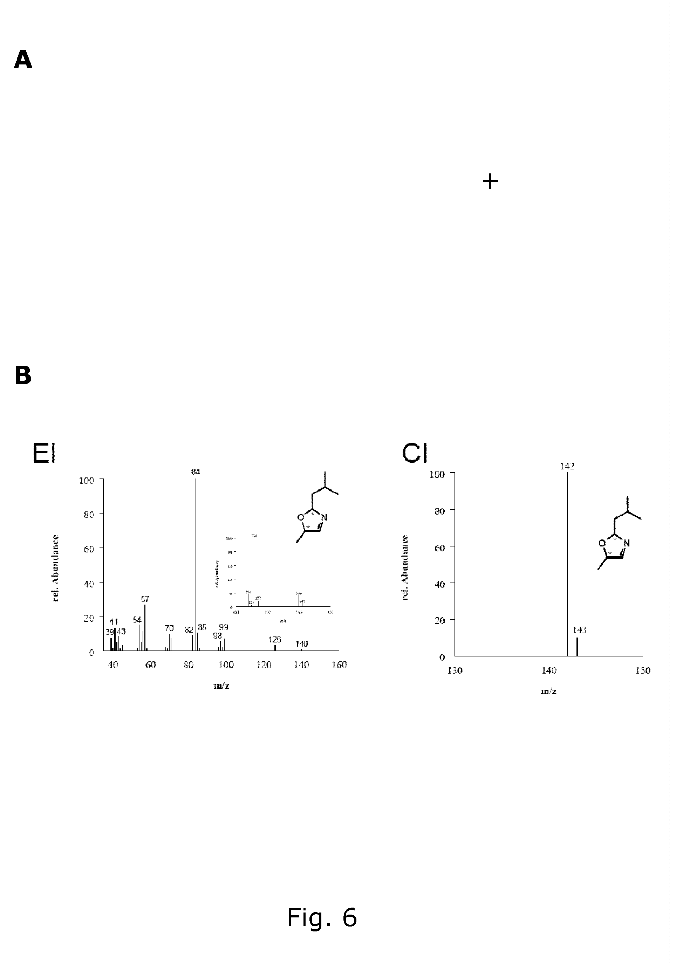

Figure 6 shows A) the synthesis of 2-(2-methylpropyl)-5-methyl-3-oxazoline and B) the mass spectra of 2-(2-methylpropyl)-5-methyl-3-oxazoline.

### Figure 7

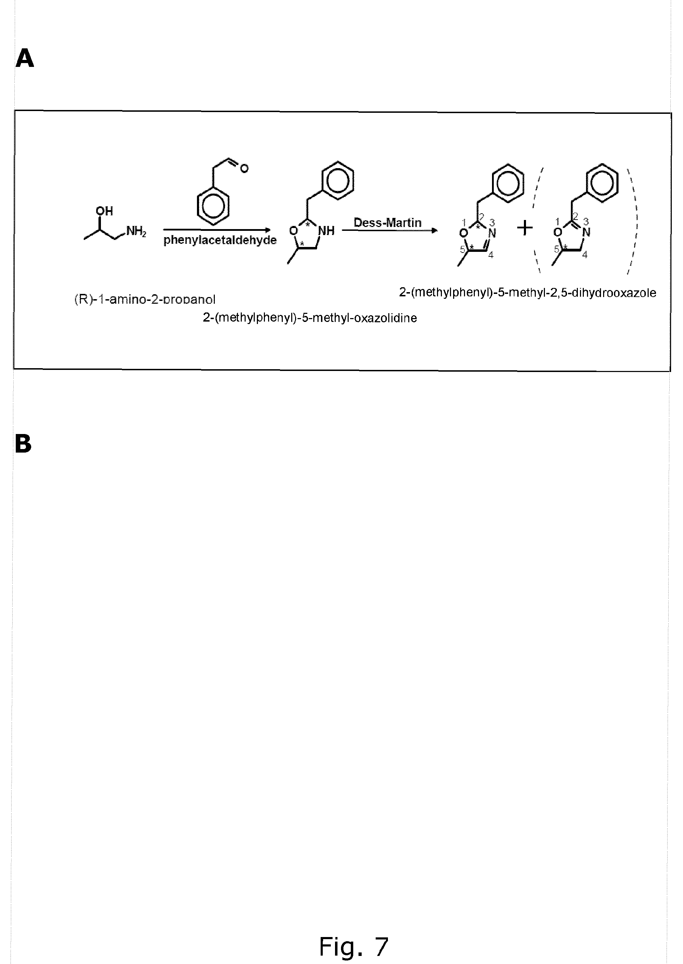

Figure 7A shows A) the synthesis of 2-methylphenyl-5-methyl-3-oxazoline and B) the mass spectra of 2-methylphenyl-5-methyl-3-oxazoline.

### Figure 8

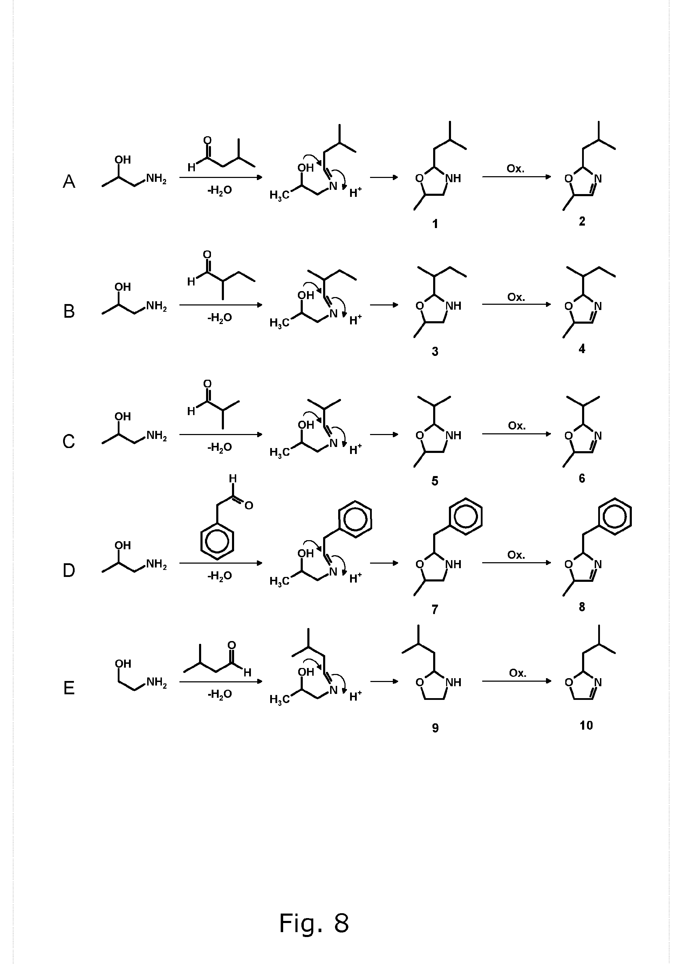

Figure 8 shows synthetic routes used in the preparation of 2-substituted-5-methyl-3-oxazolines. A: 2-(2-methylpropyl)- (2), B: 2-(1-methylpropyl)- (4), C: 2-(1-methylethyl)- (6) and D: 2-(methylphenyl)-5-methyl-3-oxazoline (8). E: 2-(2-methylpropyl)-3-oxazoline (10).

The present invention will now be described in more detail in the following.

### Detailed description of the invention

The present invention relates to novel stable aroma precursors (in dry form) that can rapidly release key aroma molecules in the presence of water and/or hydrolytic enzymes. Thus, controlled aroma release under mild conditions just prior to or during the consumption event may be obtained. It also permits increasing aroma freshness and the characteristic authentic aroma of a given food or beverage. Aroma release may take place in the food preparation phase or even during the consumption event in the mouth.

### Compounds

In its most general aspect the invention relates to compounds of the formula 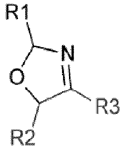 and/or 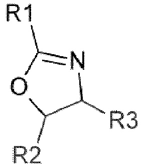 wherein
- R1 is selected from the group consisting of hydrogen, a hydrocarbon, a thiohydrocarbon and an aminohydrocarbon; and
- R2 and R3 are, independent from each other and selected from the group consisting of a hydrogen, a hydrocarbon, a carbonyl, a hydroxycarbonyl, a polyol, and aminohydrocarbon, or R2 is being linked to R3 by a bridge member Yₙ, thereby forming one or more rings; wherein Yₙ is being selected from the group consisting of a bond, C1-12 alkyl, aryl, a carbocyclic moiety, a heterocyclic moiety and a heteroaromatic moiety.

Such compounds may be used in any of the aspects according to the present invention.

In a general embodiment the compound is not a compound selected from the group consisting of 2,5-Dihydro-2-methyl-oxazole, 4,5-Dihydro-2-(1-methylethyl)-oxazole, 4,5-Dihydro-2-(1-methylpropyl)-oxazole, 4,5-Dihydro-2-(2-methylpropyl)-oxazole, 4,5-Dihydro-2-(phenylmethyl)-oxazole, 2,5-Dihydro-2,4-dimethyl-oxazole, 4,5-Dihydro-2,4-dimethyl-oxazole, 2,5-Dihydro-4-methyl-2-(1-methylethyl)-oxazole, 4,5-Dihydro-4-methyl-2-(1-methylethyl)-oxazole, 4,5-Dihydro-4-methyl-2-(phenylmethyl)-oxazole, 2,5-Dihydro-4-methyl-2-(phenylmethyl)-oxazole, 2,5-Dihydro-2,5-dimethyl-oxazole, 4,5-Dihydro-2,5-dimethyl-oxazole, 2,5-Dihydro-5-methyl-2-(1-methylethyl)-oxazole, 4,5-Dihydro-5-methyl-2-(1-methylethyl)-oxazole, 4,5-Dihydro-5-methyl-2-(phenylmethyl)-oxazole, 2,5-Dihydro-2,4,5-trimethyl-oxazole, 4,5-Dihydro-2,4,5-dimethyl-oxazole, 2,5-Dihydro-4,5-dimethyl-2-(1-methylethyl)-oxazole, 2,5-Dihydro-4,5-dimethyl-2-(2-methylpropyl)-oxazole, 4,5-Dihydro-4,5-dimethyl-2-(phenylmethyl)-oxazole, 4-Ethyl-2,5-dihydro-2,5-dimethyl-oxazole, 4-Ethyl-2,5-dihydro-5-methyl-2-(2-methylpropyl)-oxazole, 5-Ethyl-2,5-dihydro-2,4-dimethyl-oxazole, or 5-Ethyl-2,5-dihydro-4-methyl-2-(2-methylpropyl)-oxazole, and 4,5-Dihydro-2-methyl-5-oxazolemethanol.

Thus, one aspect relates to a compound of the formula 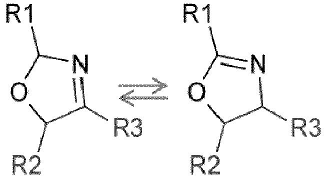 wherein
- R1 is selected from the group consisting of hydrogen, a hydrocarbon, a thiohydrocarbon and an aminohydrocarbon; and
- R2 and R3 are, independent from each other and selected from the group consisting of a hydrogen, a hydrocarbon, a carbonyl, a hydroxycarbonyl, a polyol, and aminohydrocarbon, or R2 is being linked to R3 by a bridge member Yₙ, thereby forming one or more rings; wherein Yₙ is being selected from the group consisting of a bond, C1-12 alkyl, aryl, a carbocyclic moiety, a heterocyclic moiety and a heteroaromatic moiety.
wherein the compound is not, 2,5-Dihydro-2-methyl-oxazole, 4,5-Dihydro-2-(1-methylethyl)-oxazole, 4,5-Dihydro-2-(1-methylpropyl)-oxazole, 4,5-Dihydro-2-(2-methylpropyl)-oxazole, 4,5-Dihydro-2-(phenylmethyl)-oxazole, 2,5-Dihydro-2,4-dimethyl-oxazole, 4,5-Dihydro-2,4-dimethyl-oxazole, 2,5-Dihydro-4-methyl-2-(1-methylethyl)-oxazole, 4,5-Dihydro-4-methyl-2-(1-methylethyl)-oxazole, 4,5-Dihydro-4-methyl-2-(phenylmethyl)-oxazole, 2,5-Dihydro-4-methyl-2-(phenylmethyl)-oxazole, 2,5-Dihydro-2,5-dimethyl-oxazole, 4,5-Dihydro-2,5-dimethyl-oxazole, 2,5-Dihydro-5-methyl-2-(1-methylethyl)-oxazole, 4,5-Dihydro-5-methyl-2-(1-methylethyl)-oxazole, 4,5-Dihydro-5-methyl-2-(phenylmethyl)-oxazole, 2,5-Dihydro-2,4,5-trimethyl-oxazole, 4,5-Dihydro-2,4,5-dimethyl-oxazole, 2,5-Dihydro-4,5-dimethyl-2-(1-methylethyl)-oxazole, 2,5-Dihydro-4,5-dimethyl-2-(2-methylpropyl)-oxazole, 4,5-Dihydro-4,5-dimethyl-2-(phenylmethyl)-oxazole, 4-Ethyl-2,5-dihydro-2,5-dimethyl-oxazole, 4-Ethyl-2,5-dihydro-5-methyl-2-(2-methylpropyl)-oxazole, 5-Ethyl-2,5-dihydro-2,4-dimethyl-oxazole, or 5-Ethyl-2,5-dihydro-4-methyl-2-(2-methylpropyl)-oxazole, or 4,5-Dihydro-2-methyl-5-oxazolemethanol.

In the context of this invention 3-oxazoline is a synonym term for 2,5-dihydrooxazole and 2-oxazoline is a synonym term for 4,5-dihydrooxazole. In the present context it is to be understood that the compounds according to the present invention also cover tautomers, enantiomers and diastereomers of the compounds. Tautomers are isomers (structural isomers) of organic compounds that readily interconvert by a chemical reaction called tautomerization. This reaction commonly results in the formal migration of a hydrogen atom or proton, accompanied by a switch of a single bond and adjacent double bond. The concept of tautomerizations is called tautomerism. Because of the rapid interconversion, tautomers are generally considered to be the same chemical compound.

In the present context it is to be understood that the compounds according to the invention may be enantiomers, diastereomers, as well as tautemers of the compounds according to the invention. Thus, in an embodiment the compounds are enantiomers, diastereomers, or tautemers of the compounds according to the invention.

In the present context "forming a ring" means that the atoms mentioned are connected through a bond such that the ring structure is formed. The term "ring" is used synonymously with the term "cyclic".

The term "alkyl" means a saturated linear, branched or cyclic hydrocarbon group including, for example, methyl, ethyl, isopropyl, t-butyl, heptyl, dodecyl, amyl, 2-ethylhexyl, and the like. Preferred alkyls are lower alkyls, i.e. alkyls having 1 to 10 carbon atoms, such as 1, 2, 3, 4, 5, 6, 7, 8 or 9 carbon atoms. A cyclic alkyl/cycloalkyl means a saturated carbocyclic compound consisting of one or two rings, of three to eight carbons per ring, which can optionally be substituted with one or two substituents selected from the group consisting of hydroxy, lower alkyl, lower alkoxy, alkylthio, hydroxyalkyl, alkoxycarbonyl, amino, alkylamino, alkylaminocarbonyl, arylamino- carbonyl, alkylcarbonylamino and arylcarbonylamino. The alkyl group may also be understood as a heteroalkyl. A heteroalkyl is a saturated linear, branched or cyclic hydrocarbon group (including, for example, methyl, ethyl, isopropyl, t-butyl, heptyl, dodecyl, amyl, 2-ethylhexyl, and the like) wherein one or more carbon atoms are substituted for a heteroatom selected from N, O, S, and which can optionally be substituted with one or more substituents selected from the group consisting of hydroxyl, oxo, lower alkyl, lower alkoxy, lower, alkylthio, hydroxyalkyl, alkoxycarbonyl, amino, alkylamino, alkylaminocarbonyl, aryl- aminocarbonyl, alkylcarbonylamino, or arylcarbonylamino. Heteroalkyls of the present invention may be branched or unbranched or forming a ring and may range from one (1) to fifty (50) carbon atoms in length wherein 50% or less, of said carbon atoms may be substituted for N, NH, O, or S.

A cyclic heteroalkyl/heterocyclyl means a saturated cyclic compound or part of a compound, consisting of one to more rings, of three to eight atoms per ring, incorporating one, two, three or four ring heteroatoms, selected from N, O or S, and which can optionally be substituted with one or two substituents selected from the group consisting of hydroxyl, oxo, lower alkyl, lower alkoxy, lower alkylthio, hydroxyalkyl, alkoxycarbonyl, amino, alkylamino, alkylaminocarbonyl, arylaminocarbonyl, alkylcarbonylamino, or arylcarbonylamino. Examples of common heterocycles of the present invention include, but are not limited to piperazine, piperidine, benzopyrans and pyranes which may thus be heterocyclic substituents as defined herein. Such substituents may also be denoted piperazino, piperidino, benzopyrano and pyrano, respectively. A further heterocycle of the present invention is thiophene.

Aryl represents a hydrocarbon comprising at least one aromatic ring, and may contain from 5 to 18, preferably from 6 to 14, more preferably from 6 to 10, and most preferably 6 carbon atoms. Typical aryl groups include phenyl, naphthyl, phenanthryl, anthracyl, indenyl, azulenyl, biphenylenyl, and fluorenyl groups. Particularly preferred aryl groups include phenyl, naphthyl and fluorenyl, with phenyl being most preferable. Hence, aryl represents a carbocyclic or heterocyclic aromatic radical comprising e.g. optionally substituted phenyl, naphthyl, pyridyl, thienyl, indolyl or furyl, preferably phenyl, naphthyl, pyridyl, thienyl, indolyl or furyl, and especially phenyl. Non-limiting examples of substituents are alkyl, alkenyl, alkoxy, and aryl.
The heterocyclic or heteroaromatic structure may have 1-3 rings, 3-8 ring members in each and 0 to 4 heteroatoms, or a heteroalkyl comprising 1 to 12 heteroatoms selected from the group consisting of N, O, S, or carbonyl, and wherein n is an integer between 1 and 12.

Surprisingly, the oxazoline compounds according to the present invention have the capacity to directly release Strecker aldehydes in the presence of an aqueous liquid (such as water) were identified on the basis of NMR experiments and MS techniques. Their structure was confirmed by synthesis as described in examples 1 and 2. Figure 8 provides further examples of synthetic routes used in the preparation of Strecker aldehyde precursors.
These novel class of precursors of Strecker aldehydes are stable in dry form (low water activity), but easily hydrolyse and liberate odour-active compounds (e.g. Strecker aldehydes), as there is no decarboxylation step required by the addition of an aqueous liquid. It has been demonstrated that the release of Strecker aldehydes from both isomers (diastereoisomers) show similar kinetics. As illustrated in example 3, about 75 % 2-(2-methylpropyl)-5-methyl-2,5-dihydrooxazole (2-(2-methylpropyl)-5-methyl-3-oxazoline) was converted to Strecker aldehyde only 5 minutes after addition of water at 37°C.

The hydrolysis may take place (i) by adding water to a food product or (ii) during food consumption in the mouth. The hydrolysis efficiency depends on the type of Maillard intermediate used, in particular on the rest R (see examples 3 to 6) as well as on the pH of the solution (examples 7 and 8). Lower hydrolysis speed can be compensated for by using hot water or longer hydrolysis time (e.g. chewing).

Strecker aldehydes are key molecules generated during food processing. However, they may also be formed in the mouth during mastication. That means there may be specific stable precursor intermediates present in food (e.g. chocolate, cereal-based products, cocoa/malt-based products) that decompose by interaction with the saliva to the corresponding Strecker aldehyde. This statement is supported by the examples 12 and 13.

### R1-group

The R1 may be formed of different hydrocarbons. Thus, in an embodiment the R1 group is an amino acid residue. In another embodiment R1 is selected from the group consisting of methyl, 1-methylethyl, 1-methylpropyl, 2-methylpropyl, 1-phenylmethyl, 2-methylthioethyl, 3-aminopropyl, and 4-aminobutyl.

### R2/R3-groups

The R2 and R3 groups may be formed by different groups. Thus, in an embodiment R2 and R3 are, independent from each other, selected from the group consisting of a hydrogen, a hydrocarbon comprising from 1 to 10 C-atoms, such as methyl, ethyl, propyl, a carbonyl such as acetyl, a hydroxyl carbonyl such as 1,3-dihydroxy-2-oxo-propyl or an alcohols / a polyol such as 2-hydroxyethyl, 2,3-dihydroxypropyl, 2,3,4-trihydroxybutyl, hydroxymethyl, 1,2-dihydroxyethyl, 1,2,3-trihydroxypropyl, and 1,2,3,4-tetrahydroxybutyl. In another embodiment R2 and R3 are, independent from each other, comprises C1-C7, such as C1-C6, such as C1-C5, such as C1-C4, such as C1-C3, such as C1-C2, such as C1, such as C2-C7, such as C4-C7 such as C5-C7, or such as C6-C7. The example section provides different examples where R2 is methyl.

### Specific compounds

The compounds according to the invention may also be defined as specific compounds. Thus, in an embodiment the compound is selected from the group consisting of 2-(2-methylpropyl)-5-methyl-3-oxazoline, 2-(1-methylpropyl)-5-methyl-3-oxazoline, 2-(1-methylethyl)-5-methyl-3-oxazoline, 2-(methylphenyl)-5-methyl-3-oxazoline, 2-(2-methylthioethyl)-5-methyl-3-oxazoline, 2,5-dimethyl-3-oxazoline, 2-(3-aminopropyl)-5-methyl-3-oxazoline, and 2-(4-aminobutyl)-5-methyl-3-oxazoline.

In another embodiment the compounds according to the invention is selected from the group of compounds listed in the below table:

| **Compound no** | **R1** | **R2** | **R3** |
|---|---|---|---|
| 1 | 2-Methylthioethyl | H | H |
| 2 | 3-Aminopropyl | H | H |
| 3 | 4-Aminobutyl | H | H |
| 4 | 2-Methylpropyl | H | CH3 |
| 5 | 1-Methylpropyl | H | CH3 |
| 6 | 2-Methylthioethyl | H | CH3 |
| 7 | 3-Aminopropyl | H | CH3 |
| 8 | 4-Aminobutyl | H | CH3 |
| 9 | 2-Methylpropyl | CH3 | H |
| 10 | 1-Methylpropyl | CH3 | H |
| 11 | 2-Methylthioethyl | CH3 | H |
| 12 | 3-Aminopropyl | CH3 | H |
| 13 | 4-Aminobutyl | CH3 | H |
| 14 | 1-Methylpropyl | CH3 | CH3 |
| 15 | 2-Methylthioethyl | CH3 | CH3 |
| 16 | 3-Aminopropyl | CH3 | CH3 |
| 17 | 4-Aminobutyl | CH3 | CH3 |
| 18 | 1-Methylethyl | CH3 | CH2CH3 |
| 19 | 1-Methylpropyl | CH3 | CH2CH3 |
| 20 | 1-Phenylmethyl | CH3 | CH2CH3 |
| 21 | 2-Methylthioethyl | CH3 | CH2CH3 |
| 22 | 3-Aminopropyl | CH3 | CH2CH3 |
| 23 | 4-Aminobutyl | CH3 | CH2CH3 |
| 24 | 1-Methylethyl | CH2CH3 | CH3 |
| 25 | 1-Methylpropyl | CH2CH3 | CH3 |
| 26 | 1-Phenylmethyl | CH2CH3 | CH3 |
| 27 | 2-Methylthioethyl | CH2CH3 | CH3 |
| 28 | 3-Aminopropyl | CH2CH3 | CH3 |
| 29 | 4-Aminobutyl | CH2CH3 | CH3 |

Thus, in another embodiment the compound is selected from the group consisting of compound 1-29.

In a further embodiment the compound according to the invention is of the formula

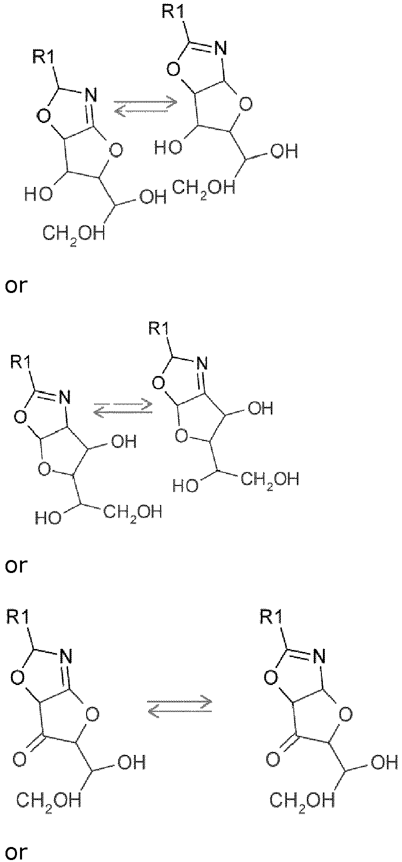

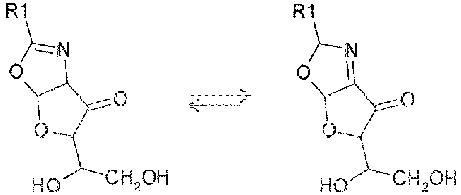

In yet a further embodiment the compound according to the invention is of the formula 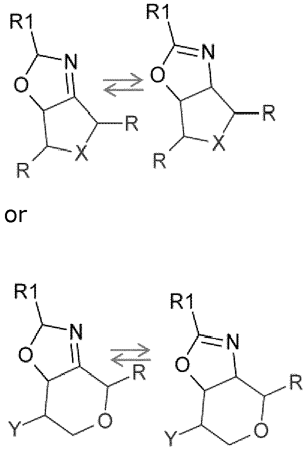 wherein
- R is hydrogen, methyl or ethyl
- XisCorO
- Y is H or OH

In yet an embodiment the compound according to the invention is of a formula selected from the group consisting of

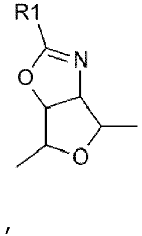

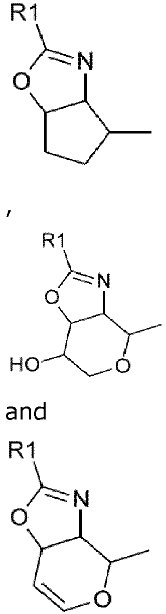

In yet another embodiment the compound according to the invention is of the formula 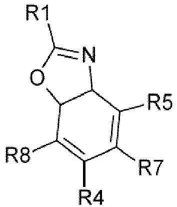 wherein R4, R5, R7 and R8 are, independent from each other, selected from the group consisting of hydrogen, oxygen, hydroxyl, cyclic or poly-cyclic hydrocarbons, heterocycles, and alcanes.

In another embodiment R4 is 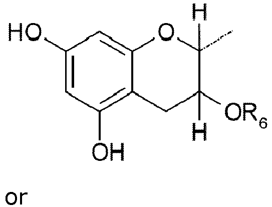 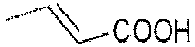
- R5 is H or OH;
- R6 is H or 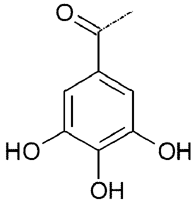
- R7 and R8 are H;
   In a further embodiment R4 is 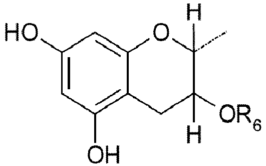 and R5, R6, R7 and R8 are H;
   or
- R4 is 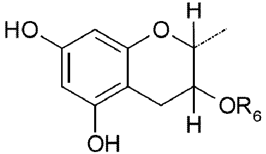
- R5, R7 and R8 are H, and
- R6 is 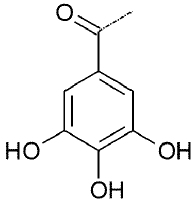 or
- R4 is 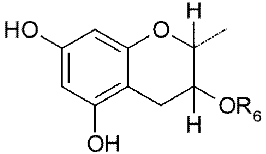
- R5 is OH, and R6, R7 and R8 are H, or
- R4 is 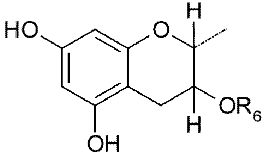
- R5 is OH, R7 and R8 are H, and
- R6 is 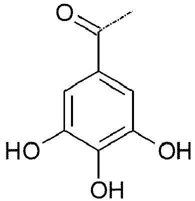 or R4 is 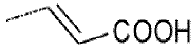 and
   R5, R7 and R8 are H.

In yet an embodiment the compounds according to the present invention is selected from the group of compounds of the formula

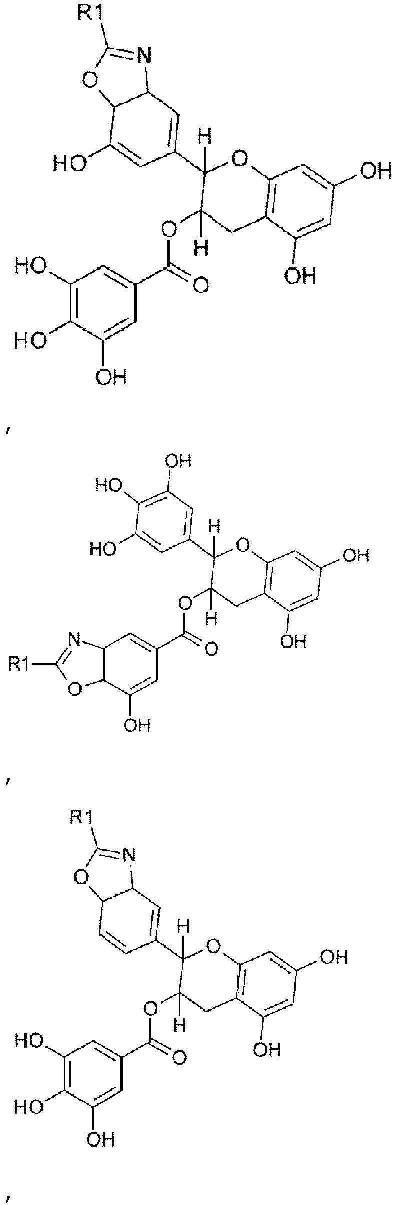

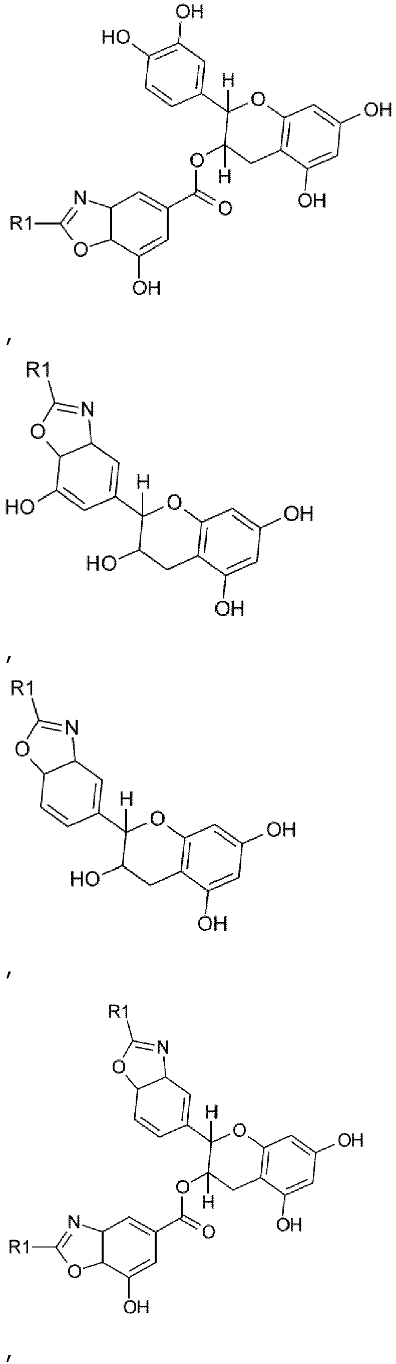

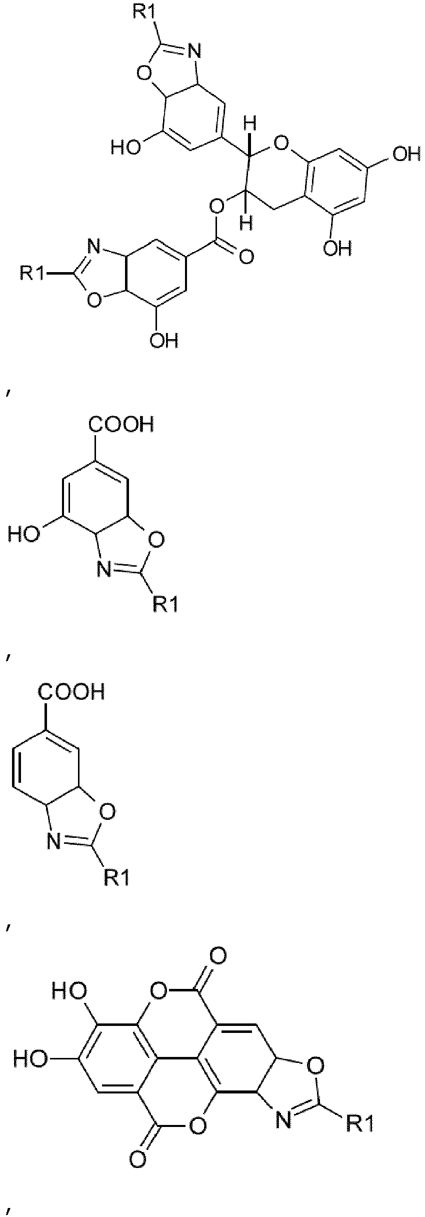

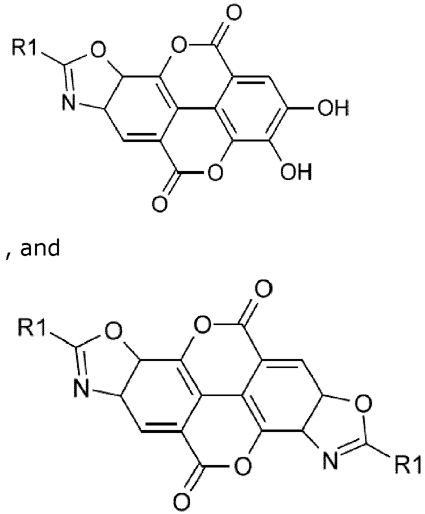

It is generally to be understood that in the indicated structural formulas that the dashed lines indicate the coupling point.

The compounds according to the invention may release Strecker aldehydes by the addition of liquid. Thus, in an embodiment the compounds according to the invention are Strecker aldehyde precursors.

### Stability

The compounds according to the present invention are stable in dry form, which may be expressed as "water activity". Water activity or a_{w} was developed to account for the intensity with which water associates with various non-aqueous constituents and solids. Simply stated, it is a measure of the energy status of the water in a system. It is defined as the vapor pressure of a liquid divided by that of pure water at the same temperature; therefore, pure distilled water has a water activity of exactly one. In an embodiment the compounds according to the present invention are stored in a dry state or in a state with low water activity. Thus, in an embodiment the compounds are stored in a state with a water activity in the range 0.01-0.7, such as in the range 0.01-0.6, such as 0.01-0.5, such as 0.01-0.4, such as 0.01-0.3, such as 0.1- 0.7, such as 0.2-0.7, such as 0.3-0.7, such as 0.4-0.7, or such as 0.5-0.7. Such low water activity may also be present if the compounds are stored in an organic solvent.

### Flavor or aroma precursor

As previously mentioned the compounds according to the present invention may function as flavor/aroma precursors. Thus, in a further embodiment the compound is a flavor/aroma precursor. In the present context the term "flavor/aroma precursor" relates to compounds which are able to liberate compounds which may be sensed both as a flavor and/or as an aroma.

### Method for obtaining the compounds according to the invention

The compounds according to the present invention may be obtained by different methods. Thus, in an aspect of the present invention relates to a method for obtaining a compound according to the present invention comprising
a) chemically synthesizing the compound; or
b) isolating or enriching a fraction of the compound from a natural source; or
c) providing the compound by fermentation of a micro-organism.

### Chemical synthesis

In a preferred embodiment the compound is synthetically produced. When the compounds are chemically synthesized, the starting materials may vary. In another embodiment the compound is synthesized from an amino acid or a Strecker aldehyde as an at least first starting material. In a further embodiment the Strecker aldehyde is selected from the group consisting of acetaldehyde, phenylacetaldehyde, 3-metylthiopropanal, 2-methylpropanal, 2-methylbutanal, 3-methylbutanal, 4-aminobutanal, and 5-aminopentanal.

In yet an embodiment the compound is synthesized from a second starting material selected from the group consisting of linear dicarbonyls, ascorbic acid, dehydroascorbic acid, cyclic enolones, oxidized phenolic compounds, polyphenols, chinones and any derivative thereof.

In another embodiment the cyclic enoles are selected from the group consisting of

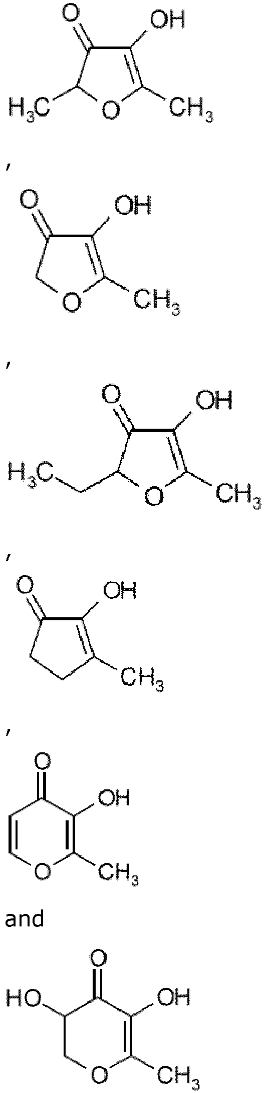

As also described above, it is to be understood that the above list of compounds also covers tautomers, enantiomers and diastereomers of the compounds. The synthesized oxazolidine may be further oxidized to the respective oxazolines according to the present invention. Thus, in a further embodiment the oxazolidines are oxidized to oxazolines. In yet a further embodiment the oxidation is a Dess-Martin oxidation.

Further details of chemical syntheses of the compounds according to the invention are presented in examples 1 and 2.

In an additional embodiment the compound is synthesized from the first starting material in combination with the second starting material.

Since the Strecker aldehydes are released in an aqueous liquid it may be an advantage to use a different solvent. Thus, in an embodiment the compound is synthesized in a non-aqueous system, such as in an organic solvent system such as in dichloromethane, propylenglycol, glycol, glycerine, triacetine, lipids (such as fats, oils, monoglycerides, diglycerides, and phospholipids).

### Isolation of compounds

The compounds according to the present invention may also be isolated from natural sources. Thus, in an embodiment the compound is isolated from a natural source selected from, for example, cocoa, cocoa beans, malt, malted cereals, roasted cereals and seeds. In an embodiment compound is isolated or enriched through thermal processing. For example, amino acids forming sensory relevant Strecker aldehydes (such as phenylalanine, leucine, valine, isoleucine) are mixed into a cocoa mass and the resulting mixture is heated at 130 to 150°C during 20 to 60 min. Then after the cocoa mass enriched with oxazolines is cooled down and ready for use.

### Production by micro-organisms

If the compounds are to be produced in micro-organisms, different organisms may be selected. Thus, in an embodiment the micro-organism are bacteria or yeast strains.

### Food ingredient

Since the compounds according to the present invention are able to liberate aroma-active aldehydes in the present of a liquid, such as water, the compounds may be incorporated in food ingredients. Thus an aspect of the present invention relates to a food ingredient enriched with one or more compounds of the formula 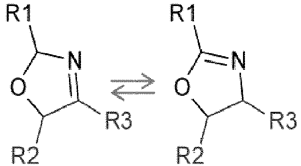 wherein
- R1 is selected from the group consisting of hydrogen, a hydrocarbon, a thiohydrocarbon and an aminohydrocarbon; and
- R2 and R3 are, independent from each other and selected from the group consisting of a hydrogen, a hydrocarbon, a carbonyl, a hydroxycarbonyl, a polyol, and aminohydrocarbon, or R2 is being linked to R3 by a bridge member Yₙ, thereby forming one or more rings; wherein Yₙ is being selected from the group consisting of a bond, C1-12 alkyl, aryl, a carbocyclic moiety, a heterocyclic moiety and a heteroaromatic moiety;
wherein the compound is not 2-methyl-3-oxazoline, 2,4-dimethyl-3-oxazoline, 2,5-dimethyl-3-oxazoline, 2,4,5-trimethyl-3-oxazoline, 5-ethyl-2,4-dimethyl-3-oxazoline, or 4-ethyl-2,5-dimethyl-3-oxazoline.

Since the aroma-active aldehydes are released in the present of moisture, it may be advantageous to have the compounds present in low moisture ingredients. Thus, in another embodiment the food ingredient has a water activity in the range 0.01-0.7, such as in the range 0.01-0.6, such as 0.01-0.5, such as 0.01-0.4, such as 0.01-0.3, such as 0.01-0.2, such as 0.01-0.1, such as 0.1- 0.7, such as 0.2-0.7, such as 0.3-0.7, such as 0.4-0.7, or such as 0.5-0.7. Preferably the water activity is below 0.3 such as below 0.2.

Depending on the specific type(s) of compound(s) which are present in the food ingredient and also depending on the type of food ingredient, the concentration may vary. Thus, in an embodiment the food ingredient has a total concentration of one or more of the compounds in the range of 0.1 ppb to 10000 ppm.

In an embodiment the food ingredient is selected from the group consisting of dry coffee powder, toppings, coatings, cocoa powder, malt, roasted/toasted cereals, and reaction flavours (as such or part of coatings).

### Food products

The compounds according to the present invention may also be part of food products which are ready to consumption, or food products which are ready to be consumed after addition of a liquid. Thus, a further aspect of the present invention relates to a food product enriched with one or more compounds of the formula 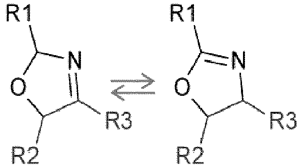 wherein
- R1 is selected from the group consisting of hydrogen, a hydrocarbon, a thiohydrocarbon and an aminohydrocarbon; and
- R2 and R3 are, independent from each other and selected from the group consisting of a hydrogen, a hydrocarbon, a carbonyl, a hydroxycarbonyl, a polyol, and aminohydrocarbon, or R2 is being linked to R3 by a bridge member Yₙ, thereby forming one or more rings; wherein Yₙ is being selected from the group consisting of a bond, C1-12 alkyl, aryl, a carbocyclic moiety, a heterocyclic moiety and a heteroaromatic moiety;
wherein the compound is not 2-methyl-3-oxazoline, 2,4-dimethyl-3-oxazoline, 2,5-dimethyl-3-oxazoline, 2,4,5-trimethyl-3-oxazoline, 5-ethyl-2,4-dimethyl-3-oxazoline, or 4-ethyl-2,5-dimethyl-3-oxazoline;
or
a food product enriched with a food ingredient according to the present invention. Thus, the compounds may be added directly to the food product or be present through the presence of a food ingredient comprising the one or more compounds.

Similar to the food ingredient, the food product preferably have a low moisture content. Thus, in an embodiment the food product has a water activity in the range 0.01-0.7, such as in the range 0.01-0.6, such as 0.01-0.5, such as 0.01-0.4, such as 0.01-0.3, such as 0.01-0.2, such as 0.01-0.1, such as 0.1- 0.7, such as 0.2-0.7, such as 0.3-0.7, such as 0.4-0.7, or such as 0.5-0.7. Preferably the water activity is below 0.3 such as below 0.2.

Depending on the specific type(s) of compound(s) which are present in the food product and also depending on the type of food product, the concentration may vary. Thus, in yet another embodiment the food product has a total concentration of one or more of the compounds in the range of 0.1 ppb to 10000 ppm.

The specific type of food product may vary. Possible products are powders and solid foods that are reconstituted before consumption by adding (hot) milk or water, i.e. dry culinary products, coffee mixes, breakfast cereals, chocolate, etc. Alternatively, the flavor-active molecules can be liberated during consumption using products that stay relatively long in the mouth, i.e. chocolate, confectionery products, cereal-based snacks. Thus, in an embodiment the food product is selected from the group consisting of dry culinary products, beverages (coffee, coffee mixes, cocoa malt beverages, tea), breakfast cereals, chocolate, confectionery products, and cereal-based products such as snacks, infant cereals, and all family cereals, biscuits, wafers, chewing gum and petfood. It is to be understood that such food products may also be considered as a food ingredient according to the present invention in the case that such food products forms part of a composite food product.

It may be advantageous to have the compounds isolated from other parts of the food product to avoid early release of the Strecker aldehydes due to high moisture content in the remaining part of the food product. Thus, in an embodiment the compound is encapsulated in a compartment in the food product. In another embodiment the encapsulation has a water activity below 0.4, such as below 0.3, such as below 0.2. It may also be advantageous if the compound could be controllable released from the encapsulation. Thus, in yet an embodiment the encapsulation dissolves or disintegrates by the addition of an aqueous liquid. Such encapsulations may be made from different compositions. In an embodiment the encapsulation comprises a lipophilic phase. In another embodiment the encapsulation is made of a structured lipid phase comprising a polar solvent and a lipid plus an emulsifier (examples of such structured lipid phases are described in US2011189367 and W0201173035).

### Methods for producing a flavor/aroma enriched food product or food ingredient

The flavor/aroma enriched food products or food ingredients according to the present invention may be obtained by different methods. In one aspect the invention relates to a method for producing a flavor/aroma enriched food product or food ingredient comprising
a) providing a food product or food ingredient;
b) providing one or more compounds of the formula 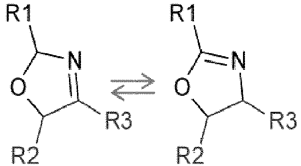 wherein
   o R1 is selected from the group consisting of hydrogen, a hydrocarbon, a thiohydrocarbon and an aminohydrocarbon; and
   o R2 and R3 are, independent from each other and selected from the group consisting of a hydrogen, a hydrocarbon, a carbonyl, a hydroxycarbonyl, a polyol, and aminohydrocarbon, or R2 is being linked to R3 by a bridge member Yₙ, thereby forming one or more rings; wherein Yₙ is being selected from the group consisting of a bond, C1-12 alkyl, aryl, a carbocyclic moiety, a heterocyclic moiety and a heteroaromatic moiety;
   wherein the compound is not 2-methyl-3-oxazoline, 2,4-dimethyl-3-oxazoline, 2,5-dimethyl-3-oxazoline, 2,4,5-trimethyl-3-oxazoline, 5-ethyl-2,4-dimethyl-3-oxazoline, or 4-ethyl-2,5-dimethyl-3-oxazoline according to any of claims 1-3; and
c) mixing a) and b.

Similar a food product may be obtained by
a) providing a food product;
b) providing a food ingredient according to the present invention;
c) mixing or assembling a) and b).

The wording "assembling" relates to the situation where the food product and food ingredient are not mixed, but instead assembled, e.g. in the case the ingredient is a topping or part of a layered product.

### Use of compound

As described previously the compounds of the present invention may function as flavor/aroma precursors. Thus, yet an aspect of the present invention relates to the use of a compound of the formula 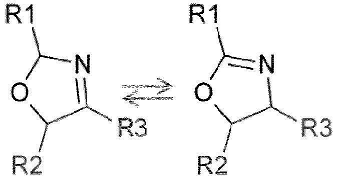 wherein
- R1 is selected from the group consisting of hydrogen, a hydrocarbon, a thiohydrocarbon and an aminohydrocarbon; and
- R2 and R3 are, independent from each other and selected from the group consisting of a hydrogen, a hydrocarbon, a carbonyl, a hydroxycarbonyl, a polyol, and aminohydrocarbon, or R2 is being linked to R3 by a bridge member Yₙ, thereby forming one or more rings; wherein Yₙ is being selected from the group consisting of a bond, C1-12 alkyl, aryl, a carbocyclic moiety, a heterocyclic moiety and a heteroaromatic moiety,
as a flavor/aroma precursor.

Yet another aspect relates to the use of a compound of the formula 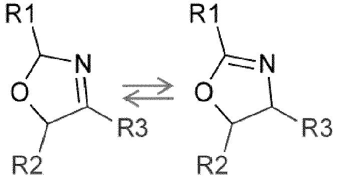 wherein
- R1 is selected from the group consisting of hydrogen, a hydrocarbon, a thiohydrocarbon and an aminohydrocarbon; and
- R2 and R3 are, independent from each other and selected from the group consisting of a hydrogen, a hydrocarbon, a carbonyl, a hydroxycarbonyl, a polyol, and aminohydrocarbon, or R2 is being linked to R3 by a bridge member Yₙ, thereby forming one or more rings; wherein Yₙ is being selected from the group consisting of a bond, C1-12 alkyl, aryl, a carbocyclic moiety, a heterocyclic moiety and a heteroaromatic moiety,

in a Strecker aldehyde release system. In an embodiment Strecker aldehyde release system is a food ingredient or a food product.

In a further aspect the invention relates to the use of a food ingredient according to the present invention as a flavor/aroma precursor in food products.

### Methods for providing Strecker aldehydes

The compounds according to the present invention may be used as precursors for the release of Strecker aldehydes. Thus, in an additional aspect the invention relates to a method for providing a Strecker aldehyde from a composition having a water activity in the range 0.01-0.7 and comprising one or more compounds of the formula 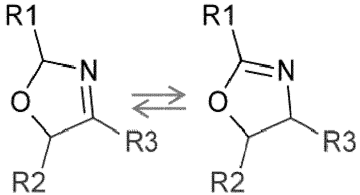 wherein
- R1 is selected from the group consisting of hydrogen, a hydrocarbon, a thiohydrocarbon and an aminohydrocarbon; and
- R2 and R3 are, independent from each other and selected from the group consisting of a hydrogen, a hydrocarbon, a carbonyl, a hydroxycarbonyl, a polyol, and aminohydrocarbon, or R2 is being linked to R3 by a bridge member Yₙ, thereby forming one or more rings; wherein Yₙ is being selected from the group consisting of a bond, C1-12 alkyl, aryl, a carbocyclic moiety, a heterocyclic moiety and a heteroaromatic moiety,
the method comprising adding an aqueous liquid to the composition. In a further embodiment the composition is a food product or a food ingredient.

The type of liquid used to initiate the release of the aldehydes may vary. Thus, in an embodiment the liquid is water, an aqueous suspension, saliva, juice or milk. In yet an embodiment the liquid comprises sugar or salts.

The temperature of the reaction may vary. Thus, in an embodiment the method is performed at a temperature in the range of 1-100°C, such as 1-40°C, such as 40-80°C or such as 80-100°C. Such temperatures may be advantageous if the product is to be consumed immediately or if the product is e.g. cereals where cold milk is used as the liquid. Thus, in yet an embodiment the temperature is in the range of 1-10°C. Since the compounds may also be present in e.g. coffee mixes which are mixed with hot water, the temperature may also be higher. Thus, in a further embodiment the temperature is in the range of 80-100°C, such as 90-98°C.
The compounds may also be released in the mouth during consumption due to the saliva. Thus, in an embodiment the temperature is in the range 30-40°C.
As previously mentioned the Strecker aldehydes may be released without a decarboxylation step. Thus, in an embodiment the method does not require decarboxylation of the one or more compounds.

### pH of reactions

The pH of the reaction may be adjusted to control the release of the Strecker aldehydes from the compounds. The reaction goes faster at lower pH's, whereas it is slowed down if the pH is raised. Thus, in an embodiment the reaction is performed at pH 4-10, such as 4-6, such as 6-8, or such as 8-10. The pH may be controlled by the components in the food product or food ingredient or by the added liquid.

It should be noted that embodiments and features described in the context of one of the aspects of the present invention also apply to the other aspects of the invention. Especially the embodiment relating to the compounds, also relates to the other aspects of the invention wherein similar compounds are included.

All patent and non-patent references cited in the present application, are hereby incorporated by reference in their entirety.

The invention will now be described in further details in the following non-limiting examples.

### Examples

The examples illustrate the release of flavour-active Strecker aldehydes and the sensory impact achieved by using such oxazoline precursors in food products.

### Example 1

Syntheses of the 3-oxazolines derived from valine, leucine, and isoleucine.

### Methods

(R)-1-amino-2-propanol (20 mmol) was dissolved in dichloromethane (80 mL; dried over anhydrous sodium sulfate), the respective aldehyde (20 mmol; methylpropanal, 3-methylbutanal, or 2-methylbutanal) was added and the reaction mixture was stirred at room temperature for 12 h (yield: approximately 50 %). To an aliquot (20 mL; about 2.5 mmol of 3-oxazolidine), first dichloromethane (60 mL), and afterwards Dess-Martin periodinane (3.5 mmol) was added. Using freshly prepared Dess-Martin periodinane, the reaction to the corresponding 3-oxazolines was completed after 30 minutes. (If the oxidation reagent is not freshly prepared, more acetic acid might be present in the reaction mixture (detectable by human nose), and thus, sodium carbonate should added for buffering.) After addition of pentane (25 mL), the mixture was filtered and submitted to high vacuum distillation at about 50 °C (SAFE technique). The obtained distillate was evaporated to a volume of approximately 1 mL (oily residue). After addition of a pentane/diethyl ether mixture (3 mL; 70/30; v/v), the solution was applied onto a column chromatography (22 x 2 cm; starting conditions: 100 % of pentane) using a diol phase (Bakerbond, Baker,Griesheim, Germany).

Leucine related precursor:
Elution was performed as following:
   ● Fraction 1: 100 mL pentane/diethyl ether (100/0; v/v)
   ● Fraction 2: 50 mL pentane/diethyl ether ( 95/5; v/v)
   ● Fraction 3: 200 mL pentane/diethyl ether ( 95/5; v/v)
   ● Fraction 4: 100 mL pentane/diethyl ether ( 95/5; v/v).

Fraction 3 contained the respective 3-oxazoline 2-(2-methylpropyl)-5-methyl-3-oxazoline (present in two diastereomeric forms) and was used for re-chromatography using the same diol phase with the following elution:
● Fraction 1: 100 mL pentane/diethyl ether (100/0; v/v)
● Fraction 2: 50 mL pentane/diethyl ether ( 95/5; v/v)
● Fraction 3: 50 mL pentane/diethyl ether ( 95/5; v/v)
● Fraction 4: 50 mL pentane/diethyl ether ( 95/5; v/v)
● Fraction 5: 50 mL pentane/diethyl ether ( 95/5; v/v).

Isomer I of 2-(2-methylpropyl)-5-methyl-3-oxazoline was found in fraction 3, a mixture of isomers I and II was found in fraction 4, and isomer II was found in fraction 5.

Figure 6A shows the synthesis of 2-(2-methylpropyl)-5-methyl-3-oxazoline and Figure 6B shows the mass spectra of 2-(2-methylpropyl)-5-methyl-3-oxazoline:
Isoleucine related precursor:
Elution was performed as following:
   ● Fraction 1: 100 mL pentane/diethyl ether (100/0; v/v)
   ● Fraction 2: 150 mL pentane/diethyl ether ( 95/5; v/v)
   ● Fraction 3: 20 mL pentane/diethyl ether ( 95/5; v/v)
   ● Fraction 4: 50 mL pentane/diethyl ether ( 95/5; v/v)
   ● Fraction 5: 100 mL pentane/diethyl ether (90/10; v/v).

Fraction 2 contained the respective 3-oxazoline 2-(1-methylpropyl)-5-methyl-3-oxazoline (single isomer I) and fraction 4 and 5 contained the second isomer. No further re-chromatography was needed.

Valine related precursor:
Elution was performed as following:
   ● Fraction 1: 100 mL pentane/diethyl ether (100/0; v/v)
   ● Fraction 2: 150 mL pentane/diethyl ether ( 95/5; v/v)
   ● Fraction 3: 100 mL pentane/diethyl ether (90/10; v/v)
   ● Fraction 4: 50 mL pentane/diethyl ether (90/10; v/v).

Fraction 3 and 4 contained the respective 3-oxazoline 2(1-methylethyl)-5-methyl-3-oxazoline (present in two diastereomeric forms). Re-chromatography using the same system as mentioned above did not differentiate both isomers.

Leucine related precursor: 2-(2-methylpropyl)-5-methyl-3-oxazoline MS-EI; m/z (%): 84 (100), 57 (27), 54 (14), 41 (13), 56 (11), 85 (10), 43 (9), 70 (9), 82 (8), 39 (7), 71 (7), 83 (7), 99 (7), 97 (6), 42 (5), 140 ([M-H]⁺; 1), 141 (M⁺; tr).
MS-CI; m/z (%): 142 ([M+H]⁺;100), 143 (11).
¹H NMR [400 MHz; CD₂Cl₂]: 0.97 [d, J = 6.7 Hz, 6H, H-C(7,8)], 1.30 [d, J = 6.7 Hz, 3H, H-C(1)], 1.42-1.48 [m, 1H, H-C(5a)], 1.57-1.63 [m, 1H, H-C(5b)], 1.83-1.92 [m, 1H, H-C(6)], 4.69-4.74 [m, 1H, H-C(2)], 5.55-5.59 [m, 1H, H-C(4)], 7.36 [d, J = 2.6 Hz, 1H, H-C(3)].
¹³C NMR [400 MHz, CD₂Cl₂]: 19.75 [C(1)], 22.78 [C(7)], 23.80 [C(8)], 25.27 [C(6)], 46.80 [C(5)], 82.07 [C(2)], 105.61 [C(4)], 7,36 [C(3)].

Isoleucine related precursor: 2-(1-methylpropyl)-5-methyl-3-oxazoline
MS-EI; m/z (%): 84 (100), 56 (48), 57 (28), 112 (28), 85 (27), 54 (16), 41 (13), 43 (10), 70 (10), 82 (10), 71 (9), 83 (9), 39 (8), 97 (7), 113 (6), 140 ([M-H]⁺; 1), 141 (M⁺; tr).
MS-CI; m/z (%): 142 ([M+H]⁺; 100), 143 (11).
¹H NMR [400 MHz; CDCl₃]: 0.92 [t, J = 7.4 Hz, 3H, H-C(7)], 1.16 [d, J = 7.0 Hz, 3H, H-C(8)], 1.30 [d, J = 6.2 Hz, 3H, H-C(1)], 1.42-1.54 [m, 1H, H-C(6a)], 1.61-1.72 [m, 1H, H-C(6b)], 2.33-2.42 [m, 1H, H-C(5)], 3.34-3.39 [m, 1H, H-C(3a)], 3.87-3.93 [m, 1H, H-C(3b)], 4,59-4,67 [m, 1H, H-C(2)].
¹³C NMR [400 MHz, CDCl₃]: 11.65 [C(7)], 13.59 [C(8)], 18.33 [C(1)], 24.42 [C(6)], 40.18 [C(5)], 81.88 [C(3)], 109.38 [C(2)], 164.08 [C(4)].

Valine related precursor: 2-(1-methylethyl)-5-methyl-3-oxazoline
MS-EI; m/z (%): 84 (100), 56 (75), 57 (63), 70 (30), 112 (30), 85 (29), 83 (28), 68 (26), 110 (15), 55 (9), 41 (12), 43 (9), 39 (8), 127 (M⁺; 1).
MS-CI; m/z (%): 128 ([M+H]⁺; 100), 129 (11).
¹H NMR [400 MHz; CDCl₃]: 0.93 [d, J = 6.8 Hz, 3H, H-C(8)], 0.96 [d, J = 6.8 Hz, 3H, H-C(9)], 1.29 [d, J = 6.7 Hz, 3H, H-C(6)], 1.91-1.99 [m, 1H, H-C(7)], 4.80-4.86 [m, 1H, H-C(5)], 5.56-5.59 [m, 1H, H-C(2)], 7.48 [d, J = 2.5 Hz, 1H, H-C(4)].
¹³C NMR [100 MHz, CDCl₃]: 16.61 [C(8 or 9)], 17.26 [C(9 or 8)], 18.26 [C(6)], 33.52 [C(7)], 82.10 [C(5)], 110.25 [C(2)], 163.87 [C(4)].

### Example 2

Syntheses of the 3-oxazolines derived from phenylalanine.

### Methods

(R)-1-amino-2-propanol (20 mmol) was dissolved in dichloromethane (200 mL; dried over anhydrous sodium sulfate), phenylacetaldehyde (20 mmol; freshly prepared by distillation) was added and the reaction mixture was stirred at room temperature for 30 min (yield: approximately 14 %). To the reaction mixture (about 2.8 mmol of 3-oxazolidine), Dess-Martin periodinane (3.5 mmol) was added. Using freshly prepared Dess-Martin periodinane, the reaction to the corresponding 3-oxazoline was completed after 30 minutes. (If the oxidation reagent is not freshly prepared, more acetic acid might be present in the reaction mixture (detectable by human nose), and thus, sodium carbonate should be added for buffering.) After addition of pentane (25 mL), the mixture was filtered and submitted to high vacuum distillation at about 50 °C (SAFE technique). The obtained distillate was evaporated to a volume of approximately 1 mL (oily residue). After addition of a pentane/diethyl ether mixture (3 mL; 70/30; v/v), the solution was applied onto a column chromatography (22 x 2 cm; starting conditions: 100 % of pentane) using a diol phase (Bakerbond, Baker).

Phenylalanine related precursor:
Elution was performed as following:
   ● Fraction 1: 100 mL pentane/diethyl ether (100/0; v/v)
   ● Fraction 2: 200 mL pentane/diethyl ether (90/10; v/v)
   ● Fraction 3: 150 mL pentane/diethyl ether (90/10; v/v)
   ● Fraction 4: 50 mL pentane/diethyl ether (90/10; v/v).

Fraction 3, containing the respective 3-oxazoline (2-methylphenyl-5-methyl-3-oxazoline), was used for re-chromatography using the same diol phase with the following elution:
● Fraction 1: 100 mL pentane/diethyl ether (100/0; v/v)
● Fraction 2: 250 mL pentane/diethyl ether (90/10; v/v)
● Fraction 3: 125 mL pentane/diethyl ether (90/10; v/v)
● Fraction 4: 100 mL pentane/diethyl ether (80/20; v/v)
● Fraction 5: 100 mL pentane/diethyl ether (80/20; v/v).

Isomer I of 2-methylphenyl-5-methyl-3-oxazoline was found in fraction 3, a mixture of isomers I and II was found in fraction 4, and isomer II was found in fraction 5.

Figure 7A shows the synthesis of 2-methylphenyl-5-methyl-3-oxazoline and figure 7B shows the mass spectra of 2-methylphenyl-5-methyl-3-oxazoline.

Phenylalanine related precursor: 2-methylphenyl-5-methyl-3-oxazoline MS-EI; m/z (%): 84 (100), 91 (70), 92 (42), 57 (39), 65 (11), 77 (11), 104 (10), 39 (8), 41 (8), 103 (8), 131 (8), 130 (8), 44 (7), 40 (6), 51 (8), 56 (8), 78 (6), 85 (5), 174 (5), 175 (M⁺; 4). MS-CI; m/z (%): 176 ([M+H]⁺; 100), 177 (12). *¹H* NMR [400 MHz; CD₂Cl₂]: 1.25 [d, J = 6.7 Hz, 3H, H-C(6)], 2.92 [dd, J = 13.8 Hz, 5.7 Hz, 1H, H-C(7a)], 3.08 [dd, J = 13.8 Hz, 5.0 Hz, H-C(7b)], 4.67-4.70 [m, 1H, H-C(5)], 5.95-5.98 [m, 1H, H-C(2)], 7.22-7.33 [m, 5H, H-C(9, 10, 11, 12, 13)], 7.40 [d, J = 2.4 Hz, 1H, H-C(4)]. *¹³*C NMR [100 MHz, CD₂Cl₂]: 18.43 [C(6)], 42.11 [C(7)], 82.39 [C(5)], 106.58 [C(2)], 126.75 [C(11)], 128.46 [C(9, 13)], 130.40 [C(10, 12)], 106.58 [C(8)], 164.70 [C(4)].

### Example 3

Release of 3-methylbutanal from 2-(2-methylpropyl)-5-methyl-3-oxazoline.

### Method

2-(2-Methylpropyl)-5-methyl-3-oxazoline (dissolved in pentane/diethyl ether; 2 mL) was added to ethanol (0.5 mL). The pentane/diethyl ether was carefully evaporated, and finally made up to 5 mL with ethanol. An aliquot (0.5 mL) was added to water (5 mL) containing the isotopically labelled standard ([²H₂]-3-methylbutanal) and stirred in closed glass vials for 5, 15 or 30 min at 37 °C. A control experiment was performed with 2-(2-methylpropyl)-5-methyl-3-oxazoline and the respective standard without adding water. For headspace measurements, the samples were directly subjected to HS-GC-MS. For liquid measurements, the samples were cooled in an ice bath and extracted with diethyl ether (3 x 15 mL; liquid-liquid extraction). The organic phases were combined, dried over anhydrous sodium sulphate, and the solvent was distilled off (20 °C, 500 mbar) to about 4 mL. An aliquot (2 µL) was used for the stable isotope dilution assays ((GC-) GC-MS).

### Results and conclusion

The results shown in Table 1 below clearly indicate substantial release of 3-methylbutanal within few minutes after addition of water.

**Table 1: Kinetics of the release of the Strecker aldehyde 3-methylbutanal from 2-(2-methylpropyl)-5-methyl-3-oxazoline^{a}**

| **Time (min)** | **3-methylbutanal (mol %) generated from^{b}** | |
|---|---|---|
| | **isomer I** | **isomer II** |
| Control^{c} | 0.3 | 0.4 |
| 5 | 72.7 | 72.5 |
| 15 | 82.4 | 81.4 |
| 30 | 91.1 | 92.2 |

| | | |
|---|---|---|
| a) The precursor was stirred in water at 37 °C. b) In relation to the amount of precursor used. c) Control experiment without addition of water. | | |

### Example 4

Release of phenylacetaldehyde from 2-methylphenyl-5-methyl-3-oxazoline.

### Method

2-Methylphenyl-5-methyl-3-oxazoline (dissolved in pentane/diethyl ether; 2 mL) was added to ethanol (0.5 mL). The pentane/diethyl ether was carefully evaporated, and finally made up to 5 mL with ethanol. An aliquot (0.5 mL) was added to water (5 mL) containing the isotopically labelled standard ([¹³C₂]-phenylacetaldehyde) and stirred in closed glass vials for 5, 15 or 30 min at 37 °C. A control experiment was performed with 2-methylphenyl-5-methyl-3-oxazoline and the respective standard without adding water. For headspace measurements, the samples were directly subjected to HS-GC-MS. For liquid measurements, the samples were cooled in an ice bath and extracted with diethyl ether (3 x 15 mL; liquid-liquid extraction). The organic phases were combined, dried over anhydrous sodium sulphate, and the solvent was distilled off (20 °C, 500 mbar) to about 4 mL. An aliquot (2 µL) was used for the stable isotope dilution assays ((GC-) GC-MS).

### Results and conclusion

Similarly to example 3, substantial release of 2-phenylacetaldehyde was observed within few minutes after addition of water (Table 2).

**Table 2: Kinetics of the release of the Strecker aldehyde phenyacetaldehyde from 2-methylphenyl-5-methyl-3-oxazoline^{a}.**

| **Time (min)** | **phenylacetaldehyde (mol %) generated from^{b}** | |
|---|---|---|
| | **isomer I** | **isomer II** |
| Control^{c} | 0.2 | 0.3 |
| 5 | 16.6 | 17.2 |
| 15 | 23.1 | 32.2 |
| 30 | 42.5 | 46.5 |

| | | |
|---|---|---|
| a) The precursor was stirred in water at 37 °C. b) In relation to the amount of precursor used. c) Control experiment without addition of water. | | |

### Example 5

Release of 2-methylbutanal from 2-(1-methylpropyl)-5-methyl-3-oxazoline.

### Methods

2-(1-Methylpropyl)--5-methyl-3-oxazoline (dissolved in pentane/diethyl ether; 2 mL) was added to ethanol (0.5 mL). The pentane/diethyl ether was carefully evaporated, and finally made up to 5 mL with ethanol. An aliquot (0.5 mL) was added to water (5 mL) containing the isotopically labelled standard ([²H₂]-2-methylbutanal) and stirred in closed glass vials for 5, 15 or 30 min at 37 °C. A control experiment was performed with 2-(1-methylpropyl)--5-methyl-3-oxazoline and the respective standard without adding water. For headspace measurements, the samples were directly subjected to HS-GC-MS. For liquid measurements, the samples were cooled in an ice bath and extracted with diethyl ether (3 x 15 mL; liquid-liquid extraction). The organic phases were combined, dried over anhydrous sodium sulphate, and the solvent was distilled off (20 °C, 500 mbar) to about 4 mL. An aliquot (2 µL) was used for the stable isotope dilution assays ((GC-) GC-MS).

### Results and conclusion

The results shown in Table 3 below indicate substantial release of 2-methylbutanal within few minutes after addition of water.

**Table 3: Kinetics of the release of the Strecker aldehyde 2-methylbutanal from 2-(1-methylpropyl)-5-methyl-3-oxazoline^{a}.**

| **Time (min)** | **2-methylbutanal (mol %) generated from^{b}** |
|---|---|
| | **isomer I / isomer II** |
| Control^{C} | 1.1 |
| 5 | 14.6 |
| 15 | 33.3 |
| 30 | 55.6 |

| | |
|---|---|
| a) The precursor was stirred in water at 37 °C. b) In relation to the amount of precursor used. c) Control experiment without addition of water. | |

### Example 6

Release of methylpropanal from 2-(1-methylethyl)-5-methyl-3-oxazoline.

### Methods

2-(1-Methylethyl)-5-methyl-3-oxazoline (dissolved in pentane/diethyl ether; 2 mL) was added to ethanol (0.5 mL). The pentane/diethyl ether was carefully evaporated, and finally made up to 5 mL with ethanol. An aliquot (0.5 mL) was added to water (5 mL) containing the isotopically labelled standard ([²H₆]-methylpropanal) and stirred in closed glass vials for 5, 15 or 30 min at 37 °C. A control experiment was performed with 2-(1-methylethyl)-5-methyl-3-oxazoline and the respective standard without adding water. The samples were directly subjected to HS-GC-MS.

### Results and conclusion

The results shown in Table 4 clearly indicate substantial release of methylpropanal within few minutes after addition of water.

**Table 4: Kinetics of the release of the Strecker aldehyde methylpropanal from 2-(1-methylethyl)-5-methyl-3-oxazoline^{a}.**

| **Time (min)** | **methylpropanal (mol %) generated from^{b}** |
|---|---|
| | **isomer I / isomer II** |
| Control^{C} | 0.1 |
| 5 | 46.0 |
| 15 | 53.5 |
| 30 | 56.1 |

| | |
|---|---|
| a) The precursor was stirred in water at 37 °C. b) In relation to the amount of precursor used. c) Control experiment without addition of water. | |

### Example 7

Impact of pH value on the kinetics of the release of 3-methylbutanal from 2-(2-methylpropyl)-5-methyl-3-oxazoline.

### Methods

2-(2-Methylpropyl)-5-methyl-3-oxazoline (dissolved in pentane/diethyl ether; 2 mL) was added to ethanol (0.5 mL). The pentane/diethyl ether was carefully evaporated, and finally made up to 5 mL with ethanol. An aliquot (0.5 mL) was added to the respective buffer solution (5 mL, pH 5, 7 or 9) containing the isotopically labelled standard ([²H₂]-3-methylbutanal) and stirred in closed glass vials for 5, 15 or 30 min at 37 °C. A control experiment was performed with 2-(2-methylpropyl)-5-methyl-3-oxazoline and the respective standard without adding the respective buffer solution. For headspace measurements, the samples were directly subjected to HS-GC-MS. For liquid measurements, the samples were cooled in an ice bath and extracted with diethyl ether (3 x 15 mL; liquid-liquid extraction). The organic phases were combined, dried over anhydrous sodium sulphate, and the solvent was distilled off (20 °C, 500 mbar) to about 4 mL. An aliquot (2 µL) was used for the stable isotope dilution assays ((GC-) GC-MS).

### Results and conclusion

The results (Table 5) indicate significantly faster release of 3-methylbutanal at pH 5 and 7 as compared to pH 9.

**Table 5: Impact of pH value on the kinetics of the release of 3-methylbutanal from 2-(2-methylpropyl)-5-methyl-3-oxazoline^{a}**

| **Time (min)** | **3-methylbutanal (mol %) generated from^{b}** | | |
|---|---|---|---|
| | **pH 5** | **pH 7** | **pH 9** |
| Control^{c} | 0.5 | 0.3 | 0.3 |
| 10 | 95.1 | 54.8 | 9.0 |
| 60 | 98.9 | 97.9 | 35.7 |

| | | | |
|---|---|---|---|
| a) The precursor was stirred in the respective buffer solution at 37 °C. b) In relation to the amount of precursor used. c) Control experiment without addition of the respective buffer solution. | | | |

### Example 8

Impact of pH value on the kinetics of the release phenylacetaldehyde from 2-methylphenyl-5-methyl-3-oxazoline.

### Methods

2-Methylphenyl-5-methyl-3-oxazoline (dissolved in pentane/diethyl ether; 2 mL) was added to ethanol (0.5 mL). The pentane/diethyl ether was carefully evaporated, and finally made up to 5 mL with ethanol. An aliquot (0.5 mL) was added to the respective buffer solution (5 mL, pH 5, 7 or 9) containing the isotopically labelled standard ([¹³C₂]-phenylacetaldehyde) and stirred in closed glass vials for 5, 15 or 30 min at 37 °C. A control experiment was performed with 2-methylphenyl-5-methyl-3-oxazoline and the respective standard without adding the respective buffer solution. For headspace measurements, the samples were directly subjected to HS-GC-MS. For liquid measurements, the samples were cooled in an ice bath and extracted with diethyl ether (3 x 15 mL; liquid-liquid extraction). The organic phases were combined, dried over anhydrous sodium sulphate, and the solvent was distilled off (20 °C, 500 mbar) to about 4 mL. An aliquot (2 µL) was used for the stable isotope dilution assays ((GC-) GC-MS).

### Results and conclusion

The results (Table 6) indicate significantly faster release of phenylacetaldehyde at pH 5 as compared to pH 7 and 9.

**Table 6: Impact of pH value on the kinetics of the release of phenylacetaldehyde from 2-methylphenyl-5-methyl-3-oxazoline^{a}**

| **Time (min)** | **phenylacetaldehyde (mol %) generated from^{b}** | | |
|---|---|---|---|
| | **pH 5** | **pH 7** | **pH 9** |
| Control^{c} | 0.4 | 0.3 | 0.3 |
| 5 | 41.2 | 7.7 | 4.9 |
| 15 | 72.1 | 18.0 | 7.1 |
| 30 | 87.7 | 30.2 | 14.3 |

| | | | |
|---|---|---|---|
| a) The precursor was stirred in the respective buffer solution at 37 °C. b) In relation to the amount of precursor used. c) Control experiment without addition of the respective buffer solution. | | | |

### Example 9

Release of 3-methylbutanal from cocoa-malt-based beverage spiked with 2-(2-methylpropyl)-5-methyl-3-oxazoline.

### Methods

Cocoa-malt-based beverage (7.5 g) was weighted into a Pyrex bottle. The product was spiked with 2-(2-methylpropyl)-5-methyl-3-oxazoline (10 mg) and the sample was then reconstituted in 100 mL of cold (10 °C) or warm (60 °C) semi-skimmed milk. Samples without addition of oxazoline were prepared analogously and used as references. The impact of the addition of the oxazoline on the intensity of malty aroma (characteristic aroma of 3-methylbutanal) was evaluated by a sensory panel. Comparative profiling procedure was conducted using an 11-point scale ranging from -5 to +5. Assessors were asked to score the sample containing oxazoline against the reference. Reference product containing no added oxazoline was arbitrary positioned on the zero of the scale.

### Results and conclusion

Irrespectively on the temperature of the milk, the samples containing oxazoline were scored significantly higher (cold milk 3.6; hot milk 3.3) in malty note as compared to reference products.

### Example 10

Release of 3-methylbutanal from coffee mix beverage spiked with 2-(2-methylpropyl)-5-methyl-3-oxazoline.

### Methods

Coffee mix powder (16 g) was weighted into a Pyrex bottle. The product was spiked with 2-(2-methylpropyl)-5-methyl-3-oxazoline (10 mg) and the sample was then reconstituted in 130 ml of hot (80 °C) water. Sample without addition of oxazoline was prepared analogously and used as reference. The impact of the addition of the oxazoline on the intensity of malty aroma (characteristic aroma of 3-methylbutanal) was evaluated by a sensory panel as described in example 9. The sample containing oxazoline were scored significantly higher (3.5) in malty note as compared to reference product. To further substantiate the sensory results, the sample was analyzed by SPME-GCxGC-TOFMS.

### Results and conclusion

The results (Table 7) indicate that the conversion of oxazoline to Strecker aldehyde is very high and reaches 77% in the analysed coffee mix sample (incubation 60 °C/15 min).

**Table 7: Amounts of 3-methylbutanal in coffee mix before and after spike with 2-(2-methylpropyl)-5-methyl-3-oxazoline.**

| | 3-methylbutanal (ug/vial) |
|---|---|
| Non-spiked reference sample | 28 |
| Sample spiked with oxazoline | 215 |
| Theoretical release from spike | 244 |
| **Yield of conversion** | **77 %** |

### Example 11

Release of 3-methylbutanal from cereal based pap spiked with 2-(2-methylpropyl)-5-methyl-3-oxazoline.

### Methods

Cereal powder (12.5 g) was weighted into a plastic dish. The product was spiked with 2-(2-methylpropyl)-5-methyl-3-oxazoline (10 mg) and the sample was then reconstituted in 100 ml of warm (60 °C) water or semi-skimmed milk. Sample without addition of oxazoline was prepared analogously and used as reference. The impact of the addition of the oxazoline on the intensity of malty aroma (characteristic aroma of 3-methylbutanal) was evaluated by a sensory panel as described in example 9.

### Results and conclusion

The samples containing oxazoline were scored higher (water 1.7; milk 1.2) in malty note as compared to reference products.

### Example 12

Release of methylpropanal, 2-methylbutanal, 3-methylbutanal, and phenylacetaldehyde from commercial chocolate.

### Methods

Commercial chocolate (50 % of cocoa content) was frozen in liquid nitrogen and ground to a powder using a mixer. An aliquote (50 g) was melted at 35 °C using a water bath and [²H₂]-3-methylbutanal as well as [¹³C₂]-phenylacetaldehyde were added. After stirring for 5 min, the chocolate was cooled to room temperature and used for all further PTR-MS experiments ("spiked chocolate").
5.0 g of spiked chocolate was weighed into an iodine flask (200 mL; sealed with a gastight septum) containing a stirring bar. The chocolate was melted at 37 °C for 5 min under stirring. Afterwards, the flask was connected to a PTR-MS instrument via a peek capillary (experiments were done at 37 °C). After defined time (details are given in the respective figures via number of cycles), water (5 mL, pH 4) or saliva (5 mL), respectively, was singly added through the septum via a syringe. As blank, methylpropanal (2.5 µg), 3-methylbutanal (6.25 µg), phenylacetaldehyde (25 µg), [²H₂]-3-methylbutanal (4.3 µg), and [¹³C₂]-phenylacetaldehyde (20 µg) were added to sunflower oil (10 g) in an iodine flask (200 mL). Following parameters are used for PTR-MS measurement: inlet temperature 120 °C, chamber temperature 80 °C, inlet flow 70 mL/min. The mass traces and the dwell time used to monitor Strecker aldehydes are shown below:

| Compound Name | Mass trace (*m*/*z*) | Dwell time (msec) |
|---|---|---|
| methylpropanal | 73 | 200 |
| 2- and 3-methylbutanal | 87 | 200 |
| [²H₂]-3-methylbutanal | 89 | 200 |
| phenylacetaldehyde | 121 | 500 |
| [¹³C₂]-phenylacetaldehyde | 123 | 500 |

The results are summarised in Figures 1 to 3.
All monitored compounds (Strecker aldehydes and their corresponding labeled internal standards) showed a similar but only a small increase in blank sample after addition of water (Figure 1). On the other hand, the release of monitored Strecker aldehydes (methylpropanal, 2- and 3-methylbutanal, and phenylacetaldehyde) from spiked chocolate clearly increased after addition of water (Figure 2) or saliva (Figure 3) at 37 °C, whereas the respective standard revealed only a marginal increase.

To further confirm the PTR-MS data, all experiments are repeated using a headspace GC-MS system. Commercial chocolate (50 % of cocoa content) was frozen in liquid nitrogen and ground to a powder using a mixer. An aliquote (25 g) was melted at 35 °C using a water bath and [²H₆]-methylpropanal, [²H₂]-3-methylbutanal as well as [¹³C₂]-phenylacetaldehyde were added. After stirring for 5 min, the chocolate was cooled to room temperature and used for all further headspace experiments ("spiked chocolate"). The newly spiked chocolate (2 g) was weighed into a headspace vial (10 mL; sealed with a gastight septum) containing a stirring bar. The chocolate was melted at 37 °C for 5 min under stirring. Then, the first injection (1 mL) was done at 37 °C used as blank sample. Afterwards, the sample was cooled to approx. 16 °C. After 30 min, saliva (2 mL) was added through the septum via a syringe, and the sample was again warmed to 37 °C under stirring. After a further 5 min, the second injection (1 mL) was done again at 37 °C ("release sample"). All experiments were done in triplicates. 2-Methylbutanal was quantified using [²H₂]-3-methylbutanal. The short summary of the result is shown below:

| **Compound Name** | **Concentration in blank sample (prior addition of saliva)** | **Concentration in "release sample" (after addition of saliva)** |
|---|---|---|
| methylpropanal | 25.4 µg/kg of chocolate | 1103 µg/kg of chocolate |
| 2-methylbutanal | 354 µg/kg of chocolate | 3009 µg/kg of chocolate |
| 3-methylbutanal | 502 µg/kg of chocolate | 4456 µg/kg of chocolate |
| phenylacetaldehyde | < LoD (due to headspace analysis) | < LoD (due to headspace analysis) |

The GC-MS data permitted to demonstrate that the high release of mass trace m/z 73 observed by the PTR-MS was due to the release of methylpropanal and a yet not identified substance, which was generated in a higher amount compared to methylpropanal.

### Example 13

Link between the release of Strecker aldehydes and the simultaneous decrease of the respective oxazoline.

### Methods

Samples were prepared as described in example 3 and 4. The results for 3-methylbutanal and 2-(2-methylpropyl)-5-methyl-3-oxazoline are summarised in Tables 8 and 9. Isomer I (Table 8) released huge amounts of 3-methylbutanal already after 5 min (73 %) increasing to > 80 % after 15 min and to > 90 % after 30 min, respectively. Interestingly, starting with purified isomer I (98 %), not only 3-methylbutanal was formed but also the corresponding isomer II (e.g., 5 % after 5 min).
For isomer II (Table 9), nearly the same aroma release occurred. Also, the same tendency for isomeration was found, however, it seems that equilibration is somewhat slower.

**Table 8: Formation of 3-methylbutanal from 2-(2-methylpropyl)-5-methyl-3-oxazoline (isomer I) in combination with the time course of isomer I in water at 37 °C^{a}_{.}**

| **Time (min)** | **3-methylbutanal (mol %) generated from isomer I^{b}** | **isomer I (unreacted) (mol %)^{b}** | **isomer II (formed) (mol %)^{b}** |
|---|---|---|---|
| Control^{c} | 0.3 | 98.1 | 1.9 |
| 5 | 72.7 | 16.2 | 5.1 |
| 15 | 82.4 | 8.4 | 10.2 |
| 30 | 91.1 | 2.9 | 4.3 |

| | | | |
|---|---|---|---|
| a) The precursor was stirred in water at 37 °C. b) In relation to the amount of precursor initially used. c) Control experiment without addition of water. | | | |

**Table 9: Formation of 3-methylbutanal from 2-(2-methylpropyl)-5-methyl-3-oxazoline (isomer II) in combination with the time course of isomer II in water at 37 °C^{a}.**

| **Time (min)** | **3-methylbutanal (mol %) generated from isomer II^{b}** | **isomer I (formed) (mol %)^{b}** | **isomer II (unreacted) (mol %)^{b}** |
|---|---|---|---|
| Control^{c} | 0.4 | 1.7 | 98.3 |
| 5 | 72.5 | 3.0 | 25.7 |
| 15 | 81.4 | 3.7 | 16.7 |
| 30 | 92.2 | 2.2 | 7.2 |

| | | | |
|---|---|---|---|
| a) The precursor was stirred in water at 37 °C. b) In relation to the amount of precursor initially used. c) Control experiment without addition of water. | | | |

The results for phenylacetaldehyde and 2-methylphenyl-5-methyl-3-oxazoline are shown in Tables 10 and 11. Again, the Strecker aldehyde was liberated from isomer I already after 5 min (17 %) increasing to > 20 % after 15 min and to > 40 % after 30 min, respectively. Interestingly, the aroma release is much slower (17 % vs. 73 % after 5 min and 43 % vs. 91 % after 30 min for isomer I, respectively) as compared to 3-methylbutanal. Again, starting with purified isomer I (98 %), not only phenylacetaldehyde but also the corresponding isomer II (e.g., 7 % after 5 min) were formed. For isomer II (Table 11), nearly the same aroma release occurred.

**Table 10: Formation of phenylacetaldehyde from 2-methylphenyl-5-methyl-3-oxazoline (isomer I) in combination with the time course of isomer I in water at 37 °C^{a}.**

| **Time (min)** | **phenylacetaldehyde (mol %) generated from isomer I^{b}** | **isomer I (unreacted) (mol %)^{b}** | **isomer II (formed) (mol %)^{b}** |
|---|---|---|---|
| Control^{c} | 0.2 | 98.0 | 2.0 |
| 5 | 16.6 | 31.8 | 7.1 |
| 15 | 23.1 | 31.1 | 6.3 |
| 30 | 42.5 | 22.3 | 5.8 |

| | | | |
|---|---|---|---|
| a) The precursor was stirred in water at 37 °C. b) In relation to the amount of precursor initially used. c) Control experiment without addition of water. | | | |

**Table 11: Formation of phenylacetaldehyde from 2-methylphenyl-5-methyl-2-oxazoline (isomer II) in combination with the time course of isomer II in water at 37 °C^{a}_{.}**

| **Time (min)** | **phenylacetaldehyde (mol %) generated from isomer II^{b}** | **isomer I (formed) (mol %)^{b}** | **isomer II (unreacted) (mol %)^{b}** |
|---|---|---|---|
| Control^{c} | 0.3 | 1.8 | 98.2 |
| 5 | 17.2 | 9.0 | 28.6 |
| 15 | 32.2 | 5.4 | 26.0 |
| 30 | 46.5 | 2.7 | 20.2 |

| | | | |
|---|---|---|---|
| a) The precursor was stirred in water at 37 °C. b) In relation to the amount of precursor initially used. c) Control experiment without addition of water. | | | |

### Example 14

Release of 2-methylbutanal, 3-methylbutanal, and phenylacetaldehyde from spiked chocolate using a model mouth.

### Methods

The release of 2- and 3-methylbutanal as well as phenylacetaldehyde from spiked chocolate (1.5 g, prepared according to example 12) using model mouth at 37 °C is shown in Figure 4. The spiked chocolate was connected to model mouth. After defined time PTR-MS was connected to the mouth, and water (1.5 mL, pH 4) was added. The details are given below via number of cycles:
● Cycle number 58: PTR-MS was connected to model mouth; piston was initially only rotating
● Cycle number 70: PTR-MS was connected to model mouth; piston is now rotating and moving up and down (simulating chewing process)
● Cycle number 145: PTR-MS was connected to model mouth; addition of water (1.5 mL); piston is still rotating and moving up and down (simulating chewing process)
● Cycle number 225: PTR-MS was disconnected from model mouth.

### Results and conclusion

The results clearly indicate release of targeted aldehydes after addition of water to the chocolate.

### Example 15

In-mouth release of 3-methylbutanal from spiked chocolate.

### Methods

The release of 3-methylbutanal from spiked chocolate (2 g, prepared according to example 12) during chewing (mouth; swallow breath) is shown in Figures 5. PTR-MS was connected to the nose space of the test person during cycle numbers 0 to 65. At given time (cycle number 35) spiked chocolate was taken into mouth, chewing was started including swallow breath.

### Results and Conclusion

The results showed clear increase of the ion m/z 87 (corresponding to the 3- and 2-methylbutanal) during chewing of the chocolate.

## Claims

1. A compound of the formula 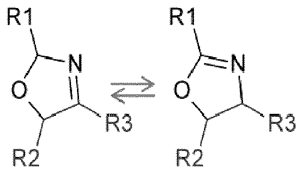 wherein
- R1 is selected from the group consisting of hydrogen, a hydrocarbon, a thiohydrocarbon and an aminohydrocarbon; and
- R2 and R3 are, independent from each other and selected from the group consisting of a hydrogen, a hydrocarbon, a carbonyl, a hydroxycarbonyl, a polyol, and aminohydrocarbon, or R2 is being linked to R3 by a bridge member Yₙ, thereby forming one or more rings; wherein Yₙ is being selected from the group consisting of a bond, C1-12 alkyl, aryl, a carbocyclic moiety, a heterocyclic moiety and a heteroaromatic moiety;
and wherein the compound is not, 2,5-Dihydro-2-methyl-oxazole, 4,5-Dihydro-2-(1-methylethyl)-oxazole, 4,5-Dihydro-2-(1-methylpropyl)-oxazole, 4,5-Dihydro-2-(2-methylpropyl)-oxazole, 4,5-Dihydro-2-(phenylmethyl)-oxazole, 2,5-Dihydro-2,4-dimethyl-oxazole, 4,5-Dihydro-2,4-dimethyl-oxazole, 2,5-Dihydro-4-methyl-2-(1-methylethyl)-oxazole, 4,5-Dihydro-4-methyl-2-(1-methylethyl)-oxazole, 4,5-Dihydro-4-methyl-2-(phenylmethyl)-oxazole, 2,5-Dihydro-4-methyl-2-(phenylmethyl)-oxazole, 2,5-Dihydro-2,5-dimethyl-oxazole, 4,5-Dihydro-2,5-dimethyl-oxazole, 2,5-Dihydro-5-methyl-2-(1-methylethyl)-oxazole, 4,5-Dihydro-5-methyl-2-(1-methylethyl)-oxazole, 4,5-Dihydro-5-methyl-2-(phenylmethyl)-oxazole, 2,5-Dihydro-2,4,5-trimethyl-oxazole, 4,5-Dihydro-2,4,5-dimethyl-oxazole, 2,5-Dihydro-4,5-dimethyl-2-(1-methylethyl)-oxazole, 2,5-Dihydro-4,5-dimethyl-2-(2-methylpropyl)-oxazole, 4,5-Dihydro-4,5-dimethyl-2-(phenylmethyl)-oxazole, 4-Ethyl-2,5-dihydro-2,5-dimethyl-oxazole, 4-Ethyl-2,5-dihydro-5-methyl-2-(2-methylpropyl)-oxazole, 5-Ethyl-2,5-dihydro-2,4-dimethyl-oxazole, 5-Ethyl-2,5-dihydro-4-methyl-2-(2-methylpropyl)-oxazole, or 4,5-Dihydro-2-methyl-5-oxazolemethanol.

2. The compound according to claim 1, wherein R1 is selected from the group consisting of methyl, 1-methylethyl, 1-methylpropyl, 2-methylpropyl, 1-phenylmethyl, 2-methylthioethyl, 3-aminopropyl, and 4-aminobutyl,

3. The compound according to claim 1 or 2, wherein the compound is selected from the group consisting of 2-(2-methylpropyl)-5-methyl-3-oxazoline, 2-(1-methylpropyl)-5-methyl-3-oxazoline, 2-(1-methylethyl)-5-methyl-3-oxazoline, 2-(methylphenyl)-5-methyl-3-oxazoline, 2-(2-methylthioethyl)-5-methyl-3-oxazoline, 2,5-dimethyl-3-oxazoline, 2-(3-aminopropyl)-5-methyl-3-oxazoline, and 2-(4-aminobutyl)-5-methyl-3-oxazoline.

4. A method for obtaining a compound according to any of claims 1-3 comprising
a) chemically synthesizing the compound; or
b) isolating or enriching a fraction of the compound from a natural source;
or
c) providing the compound by fermentation of a micro-organism.

5. The method according to claim 4, wherein the compound is synthesized from an amino acid or a Strecker aldehyde as an at least first starting material.

6. The method according to claims 4 or 5, wherein the compound is synthesized from a second starting material selected from the group consisting of linear dicarbonyls, ascorbic acid, dehydroascorbic acid, cyclic enolones, oxidized phenolic compounds, polyphenols, chinones and any derivative thereof.

7. A food ingredient enriched with one or more compounds of the formula 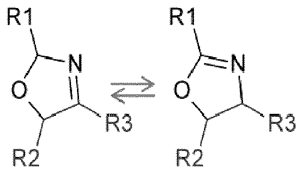 wherein
- R1 is selected from the group consisting of hydrogen, a hydrocarbon, a thiohydrocarbon and an aminohydrocarbon; and
- R2 and R3 are, independent from each other and selected from the group consisting of a hydrogen, a hydrocarbon, a carbonyl, a hydroxycarbonyl, a polyol, and aminohydrocarbon, or R2 is being linked to R3 by a bridge member Yₙ, thereby forming one or more rings; wherein Yₙ is being selected from the group consisting of a bond, C1-12 alkyl, aryl, a carbocyclic moiety, a heterocyclic moiety and a heteroaromatic moiety;
wherein the compound is not 2-methyl-3-oxazoline, 2,4-dimethyl-3-oxazoline, 2,5-dimethyl-3-oxazoline, 2,4,5-trimethyl-3-oxazoline, 5-ethyl-2,4-dimethyl-3-oxazoline, or 4-ethyl-2,5-dimethyl-3-oxazoline.

8. A food product enriched with one or more compounds of the formula 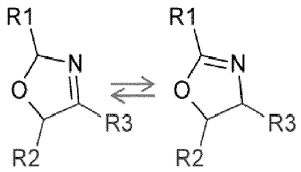 wherein
- R1 is selected from the group consisting of hydrogen, a hydrocarbon, a thiohydrocarbon and an aminohydrocarbon; and
- R2 and R3 are, independent from each other and selected from the group consisting of a hydrogen, a hydrocarbon, a carbonyl, a hydroxycarbonyl, a polyol, and aminohydrocarbon, or R2 is being linked to R3 by a bridge member Yₙ, thereby forming one or more rings; wherein Yₙ is being selected from the group consisting of a bond, C1-12 alkyl, aryl, a carbocyclic moiety, a heterocyclic moiety and a heteroaromatic moiety;
wherein the compound is not 2-methyl-3-oxazoline, 2,4-dimethyl-3-oxazoline, 2,5-dimethyl-3-oxazoline, 2,4,5-trimethyl-3-oxazoline, 5-ethyl-2,4-dimethyl-3-oxazoline, or 4-ethyl-2,5-dimethyl-3-oxazoline;
or
a food product enriched with a food ingredient according to claim 7.

9. The food product according to claim 8, wherein the compound is encapsulated in a compartment in the food product.

10. A method for producing a flavor/aroma enriched food product or food ingredient comprising
a) providing a food product or food ingredient;
b) providing one or more compounds of the formula 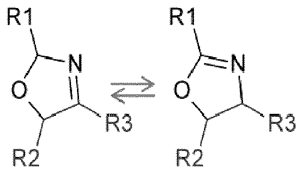 wherein
o R1 is selected from the group consisting of hydrogen, a hydrocarbon, a thiohydrocarbon and an aminohydrocarbon; and
o R2 and R3 are, independent from each other and selected from the group consisting of a hydrogen, a hydrocarbon, a carbonyl, a hydroxycarbonyl, a polyol, and aminohydrocarbon, or R2 is being linked to R3 by a bridge member Yₙ, thereby forming one or more rings; wherein Yₙ is being selected from the group consisting of a bond, C1-12 alkyl, aryl, a carbocyclic moiety, a heterocyclic moiety and a heteroaromatic moiety;
wherein the compound is not 2-methyl-3-oxazoline, 2,4-dimethyl-3-oxazoline, 2,5-dimethyl-3-oxazoline, 2,4,5-trimethyl-3-oxazoline, 5-ethyl-2,4-dimethyl-3-oxazoline, or 4-ethyl-2,5-dimethyl-3-oxazoline according to any of claims 1-3; and
c) mixing a) and b.

11. Use of a compound of the formula 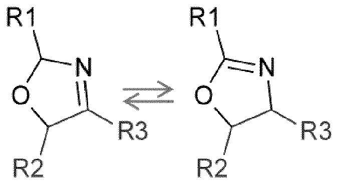 wherein
- R1 is selected from the group consisting of hydrogen, a hydrocarbon, a thiohydrocarbon and an aminohydrocarbon;
- R2 and R3 are, independent from each other and selected from the group consisting of a hydrogen, a hydrocarbon, a carbonyl, a hydroxycarbonyl, a polyol, and aminohydrocarbon, or
- R2 is being linked to R3 by a bridge member Yₙ, thereby forming one or more rings; wherein Yₙ is being selected from the group consisting of a bond, C1-12 alkyl, aryl, a carbocyclic moiety, a heterocyclic moiety and a heteroaromatic moiety,
as a flavor/aroma precursor.

12. Use of a compound of the formula 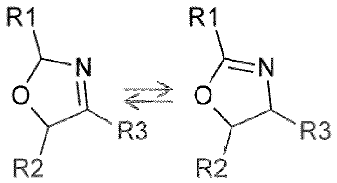 wherein
- R1 is selected from the group consisting of hydrogen, a hydrocarbon, a thiohydrocarbon and an aminohydrocarbon;
- R2 and R3 are, independent from each other and selected from the group consisting of a hydrogen, a hydrocarbon, a carbonyl, a hydroxycarbonyl, a polyol, and aminohydrocarbon, or
- R2 is being linked to R3 by a bridge member Yₙ, thereby forming one or more rings; wherein Yₙ is being selected from the group consisting of a bond, C1-12 alkyl, aryl, a carbocyclic moiety, a heterocyclic moiety and a heteroaromatic moiety,
in a Strecker aldehyde release system.

13. A method for releasing a Strecker aldehyde from a composition having a water activity in the range of 0.01-0.7 and comprising one or more compounds of the formula 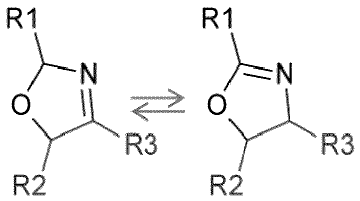 wherein
- R1 is selected from the group consisting of hydrogen, a hydrocarbon, a thiohydrocarbon and an aminohydrocarbon; and
- R2 and R3 are, independent from each other and selected from the group consisting of a hydrogen, a hydrocarbon, a carbonyl, a hydroxycarbonyl, a polyol, and aminohydrocarbon, or R2 is being linked to R3 by a bridge member Yₙ, thereby forming one or more rings; wherein Yₙ is being selected from the group consisting of a bond, C1-12 alkyl, aryl, a carbocyclic moiety, a heterocyclic moiety and a heteroaromatic moiety;
the method comprising adding an aqueous liquid to the composition.

14. The method according to claim 13, wherein the composition is a food product or a food ingredient.

15. The method according to claims 13 or 14, wherein the water activity of the food product is in the range of 0.01-0.6, such as 0.01-0.5, such as 0.01-0.4, such as 0.01-0.3, such as 0.01-0.2, such as 0.01-0.1, such as 0.1- 0.7, such as 0.2-0.7, such as 0.3-0.7, such as 0.4-0.7, or such as 0.5-0.7.
